# FASCICULE DE BREVET EUROPEEN

(11) **EP 2 996 801 B1**
(45) Date de publication et mention de la délivrance du brevet: **01.07.2020**
(21) Numéro de dépôt: 14719037.5
(22) Date de dépôt: 04.03.2014
(51) Int. Cl.: B01J 20/28, A61K 38/00, B01J 20/04, B01J 20/281, B01J 20/30, C07K 1/16, A61K 47/02

(54) **COMPOSITION À BASE D'UNE POUDRE D'HYDROXYAPATITE POUR LE TRAITEMENT D'UN LYMPHOME B OU T**
ZUSAMMENSETZUNG AUF BASIS EINES HYDROXYAPATITPULVERS ZUR BEHANDLUNG VON B- ODER T-LYMPHOM
COMPOSITION BASED ON A HYDROXYAPATITE POWDER FOR THE TREATMENT OF LYMPHOMA B OR T

(30) Priorité: 15.05.2013 FR 1354361
(43) Date de publication de la demande: 23.03.2016
(73) Titulaire: Urodelia, 31470 Saiguede (FR)
(72) Inventeur: ROUQUET, Nicole, 31400 Toulouse (FR); FRAYSSINET, Patrick, 31470 Saint-lys (FR)
(74) Mandataire: Plasseraud IP
(86) Numéro de dépôt international: PCT/FR2014/050478
(87) Numéro de publication internationale: WO 2014/184453

(56) Documents cités:
- EP-A1- 0 337 123
- WO-A2-2006/122914
- FR-A1- 2 834 653
- GB-A- 2 487 864
- I-MING HUNG ET AL: "The Properties of Sintered Calcium Phosphate with [Ca]/[P] = 1.50", INTERNATIONAL JOURNAL OF MOLECULAR SCIENCES, vol. 13, no. 10, 22 octobre 2012 (2012-10-22), pages 13569-13586, XP055101424, DOI: 10.3390/ijms131013569
- DANIEL R. CIOCCA ET AL: "A pilot study with a therapeutic vaccine based on hydroxyapatite ceramic particles and self-antigens in cancer patients", CELL STRESS AND CHAPERONES, vol. 12, no. 1, mars 2007 (2007-03), pages 33-43, XP055131849, ISSN: 1355-8145, DOI: 10.1379/CSC-218R.1

## Description

### Champ d'application :

Le domaine de l'invention est celui des traitements par immunothérapie, en particulier des traitements des cancers.

Plus précisément, l'invention a trait à des traitements autologues par immunostimulation.

Plus précisément encore, l'invention concerne des moyens de lutte notamment contre le cancer, comprenant des médicaments anti-tumoraux, de préférence des vaccins, obtenus à partir des antigènes tumoraux des patients.

La présente invention vise notamment un médicament, par exemple un vaccin antitumoral, à base d'hydroxyapatite et/ou de phosphate tricalcique.

Est également décrit un procédé de préparation d'une poudre d'hydroxyapatite et/ou de phosphate tricalcique en vue de son utilisation en tant que médicament, un procédé de préparation du médicament à base de la poudre d'hydroxyapatite et/ou de phosphate tricalcique ainsi qu'un kit de traitement comprenant la poudre d'hydroxyapatite et/ou de phosphate tricalcique.

### Etat de la technique :

Il est bien connu que plusieurs formes de traitement contre le cancer par immunothérapie existent :
- *l'administration de substances pharmacologiques* (telles que interleukine, interféron) qui augmentent la réponse immunitaire de manière non spécifique. Ces thérapies ont un degré variable de toxicité et bien qu'ils puissent montrer une certaine efficacité, beaucoup de patients ainsi traités répondent faiblement. Ces thérapies provoquent une amplification de nombreuses fonctions immunitaires ;
- *l'utilisation d'anticorps spécifiques contre les antigènes tumoraux présents à la surface des cellules tumorales.* Ces anticorps sont généralement des anticorps hybrides humanisés. Cependant, l'efficacité de ces traitements est limitée en raison de la variabilité antigénique des cellules tumorales car certaines cellules tumorales au moment du diagnostic expriment les antigènes d'intérêt et d'autres non;
- *la stimulation des cellules T.* Cette forme de thérapie est en rapport avec l'objet de l'invention. La stimulation des cellules T peut être :
   - adoptive (prélèvement de cellules T antitumorales, augmentation *in vitro* de leur nombre puis administration au patient), ou
   - active (mise en contact *in vitro* ou *in vivo* des antigènes tumoraux avec les macrophages ou des cellules apparentées pour que les cellules présentatrices d'antigènes (APC) stimulent les lymphocytes T). Ces agents stimulant les lymphocytes T sont assimilables à des vaccins thérapeutiques.

Il existe plusieurs stratégies pour générer des vaccins stimulant le système T sous forme active et spécifique. Une de ces stratégies d'immunothérapie repose sur l'emploi de protéines du choc thermique comme adjuvant. Un adjuvant a pour fonction de faire réagir le système immunitaire à une molécule à laquelle il ne réagirait pas forcément ou au moins pas aux doses employées. Il déclenche une réaction au corps étranger amenant un grand nombre de cellules présentatrices de l'antigène (APCs) au site d'injection qui vont ensuite informer les lignées lymphocytaires des molécules qu'elles doivent identifier et éliminer. Cette information se fait par exposition des molécules contre lesquelles s'immuniser sur la surface des APCs et par la synthèse par celles-ci de diverses cytokines et interleukines ainsi que des co-facteurs membranaires. Ainsi, pour générer des vaccins stimulant le système T via les protéines du choc thermique comme adjuvant, on extrait de la tumeur des protéines du choc thermique (HSPs) comme la gp 96, ou l'HSP 70. Ces protéines sont des chaperonnes et s'associent à des nombreux peptides dont les peptides (antigènes) spécifiques de la tumeur de chaque patient. Elles constituent ainsi une empreinte moléculaire de la tumeur à éradiquer et diffèrent d'un patient à un autre et d'une tumeur à l'autre. Pour le même patient elle varie également au cours du développement de la tumeur en raison de l'instabilité génétique des cellules cancéreuses.

Cette stratégie impose de purifier les protéines vaccinantes à partir de chaque tumeur contre laquelle elles doivent immuniser le patient. Le protocole de purification est long, difficilement industrialisable et sujet à de multiples contaminations par endotoxines. Il est classique de purifier les HSPs à partir d'un broyat tumoral qui est soumis à une série de centrifugation, précipitation, chromatographie sur Con A, analyse électrophorétique, et chromatographie sur Mono Q FPLC.

Les brevets US Nos 6,447,781, 6,436,404, 6,410,028, 6,383,494 et 6,030,618 décrivent des méthodes de purification de protéines HSP consistant à mettre en œuvre des colonnes chromatographiques de Con A SEPHAROSE. Cependant, ces méthodes sont perfectibles.

Par ailleurs, depuis les travaux de Tizelius en 1973, on sait que les particules d'hydroxyapatite (HAP) peuvent être utilisées pour purifier des protéines à partir de solutions complexes. Les poudres d'HAP sont utilisées comme lit fixe dans des colonnes de chromatographie à travers lesquelles est percolée la solution contenant la ou les molécules à purifier. Ces molécules se fixent sur la surface des particules de poudre d'HAP, d'où elles sont désorbées à l'aide de concentrations diverses de solutions salines telles que des tampons phosphates ou bien du chlorure de sodium, ou de calcium ce qui permet de séparer les différentes molécules de la solution par des gradients de tampons phosphate ou autres.

La demande de brevet WO 2006/122914 décrit la capacité de ces particules d'HAP pour purifier les protéines du choc thermique à partir d'extraits tissulaires. Comme énoncé ci-dessus, la purification des protéines du choc thermique à partir d'une biopsie tumorale permet de disposer de peptides et de protéines spécifiques de la tumeur. Ces peptides et protéines varient non seulement d'un patient à l'autre mais également tout au long du développement de la tumeur chez le même individu du fait de l'instabilité génique de la cellule cancéreuse. WO 2006/122914 décrit plus particulièrement un procédé de préparation en une seule étape d'antigènes tumoraux destiné à les mettre sous une forme reconnaissable par le système immunitaire afin de pouvoir l'appliquer à grande échelle par un personnel n'ayant pas une qualification de biochimiste. On sait également de cette demande de brevet internationale que les poudres d'HAP ainsi que d'autres sels de calcium peuvent être aussi utilisés comme adjuvants de vaccination et que la poudre d'hydroxyapatite ayant adsorbé les antigènes tumoraux spécifiques d'une tumeur peut être utilisée en tant que médicament contre ladite tumeur. L'Article de Ciocca et al., "A pilot study with a therapeutic vaccine based on hydroxyapatite ceramic particles and self-antigens in cancer patient", Cell stress & chaperones vol.12.no.1 (2007), pages 33-43 divulgue également la purification d'antigènes tumoraux spécifiques d'une tumeur par absorption sur une poudre d'hydroxyapatite qui est ensuite utilisée en tant que médicament contre ladite tumeur.

Ainsi, la même poudre d'HAP peut servir à la fois à purifier les protéines spécifiques d'une tumeur et stimuler le système immunitaire contre ces dernières lorsque poudres et protéines sont injectées ensembles. Cependant, les procédés de préparation des poudres d'HAP et les poudres elles-mêmes sont perfectibles.

L'utilisation de poudres dans ce but présente de nombreuses contraintes.

Les poudres doivent présenter une grande surface spécifique afin de fixer une quantité de protéine importante.

Elles doivent avoir une coulabilité importante afin de pouvoir être injectées par des aiguilles d'injection et doivent avoir une résistance suffisante pour ne pas colmater les colonnes de chromatographie. De plus, elles ne doivent pas colmater lorsqu'une solution de protéines contenant en outre de la fibrine polymérisant avec du calcium (présent dans l'HAP) est mise à percoler dans ladite colonne de chromatographie.

En plus de ces contraintes mécaniques, les propriétés de surface spécifique doivent permettre la fixation de protéines, en particulier les protéines du choc thermique et plus particulièrement encore gp96 et Hsp70. Enfin, les poudres ayant fixé les protéines spécifiques d'une tumeur donnée, particulièrement celles du choc thermique, ne doivent pas entraîner, après injection, de réactions tissulaires gênantes, ni d'effet secondaire systémique.

### Brève description de l'invention

L'invention est définie par les revendications.

Pour atteindre ces objectifs d'amélioration, les inventeurs ont eu le mérite de développer un procédé de production d'une poudre d'hydroxyapatite et/ou de phosphate tricalcique perfectionné.

La présente invention concerne donc une poudre d'hydroxyapatite et/ou de phosphate tricalcique, caractérisée en ce qu'elle a subi au moins une étape de frittage à une température comprise entre 400°C et 600°C.L'invention concerne une poudre d'HAP et/ou de phosphate tricalcique ayant les caractéristiques suivantes :
- une granulométrie G telle que G ≤ 200µm, de préférence 0 < G ≤ 25µm ou comprise entre 25µm et 45µm, et
- une surface spécifique SS telle que SS ≥30 m²/g.

Dans un mode de réalisation particulier, la poudre peut en outre présenter un rapport de la largeur à mi-hauteur sur la hauteur totale de la raie sur un spectre de diffraction des rayons X, en se plaçant à une valeur d'angle en 2θ égale à environ 31.773 degrés +/- 1%, reportée en pourcentage, comprise entre 0,2% et 0,35%, de préférence égale à 0,3% +/- 10%.

Est également décrit ici un procédé de production d'une poudre d'hydroxyapatite et/ou de phosphate tricalcique caractérisé en ce qu'il comprend au moins une étape de frittage d'une poudre d'hydroxyapatite et/ou de phosphate tricalcique à une température comprise entre 400°C et 600°C.

La divulgation concerne également un procédé de préparation d'une composition caractérisé en ce qu'il comprend les étapes suivantes :
a) la préparation d'un échantillon tumoral,
b) l'extraction des protéines cytoplasmiques de l'échantillon tumoral,
c) le passage des protéines extraites à l'étape b) au travers d'une colonne de chromatographie contenant une poudre d'hydroxyapatite et/ou de phosphate tricalcique telle que définie ci-dessus,
d) éventuellement au moins un lavage de la poudre à l'issue de l'étape c).

L'étape d'extraction des protéines cytoplasmiques vise à extraire l'ensemble des protéines cytoplasmiques présentes dans les cellules tumorales de l'échantillon, par exemple préalablement prélevé du sujet à traiter. A l'étape c) du procédé de préparation de la composition décrite ci-dessus, la colonne de chromatographie peut être mise sous pression manuellement.

Le procédé de préparation de la composition telle que décrite ci-dessus peut comprendre en outre une étape e) de mise en suspension de la poudre issue de l'étape c) ou d) dans un liquide d'injection, sans ouvrir la colonne contenant la poudre.

La présente invention concerne la composition obtenue selon le procédé de préparation décrit ci-dessus pour son utilisation thérapeutique dans le traitement de tumeurs. Elle est utilisée comme auto-vaccin thérapeutique anti-tumoral.

La présente invention concerne la composition telle que définie ci-dessus pour son utilisation dans le traitement des lymphomes B ou T chez un sujet.

La présente invention concerne également des compositions telles que décrites ci-dessus pour leur utilisation thérapeutique en association avec un second agent thérapeutique, de préférence un agent anti-tumoral et/ou un agent radio-thérapeutique, par exemple un ou plusieurs des composés suivants et/ou au moins l'un de ses/leurs sels pharmaceutiquement acceptables :
- les médicaments cytotoxiques comme
   ∘ les alkylants comprenant les moutardes à l'azote (Méchlorétamine, Cyclophosphamide, Ifosfamide, Phénylalanine moutarde, chlorambucil), et
   ∘ les dérivés de l'éthylénimine (Triéthylènethiophosphoramide), Alkylsulfonates (Busulfan), Nitrosourées (Cyclohexyl chloroéthyl nitrosourée, 1,3 bis-(2-chloroéthyl nitrosourée)), Streptozotocine, Triazènes (Diméthyl-triazeno-imidazolecarboxamide)
- les antimétabolites comprenant
   ∘ les analogues de l'acide folique comprenant le méthotrexate,
   ∘ les analogues de la pyrimidine comprenant le 5-fluorouracile, ou le cytosine arabinoside,
   ∘ les analogues de la purine comprenant le 6-mercaptopurine, 6-thioguanine, ou le déoxycoformycine ;

- les produits naturels ou semi-synthétiques comprenant les alacaloïdes de la pervenche (vinblastine, vincristine)
- les antibiotiques comprenant la doxorubicine, mitoxantrone, daunorubicine, bléomycine, dactinomycine, mytomycine C
- les enzymes tels que la L-aspariginase
- les épipodophyllotoxines tels que l'Etoposide, la Téniposide
- les composés divers tels que les Cis diamine dichloroplatinum, le Carboplatine, Hydroxyurée et Procarbazine.
- les anticorps_ciblant les CD20, CD33, CD52, le VEGF, l'HER-2neu, l'EGFR-1
- les radioéléments comme l'Iode 131 et le Calcium 45.Est également décrit ici un kit pour la mise en œuvre du procédé de préparation de la composition telle que définie ci-dessus, le kit comprenant la poudre d'hydroxyapatite et/ou de phosphate tricalcique selon l'invention, destinée à interagir avec l'extrait de protéines cytoplasmiques d'un échantillon tumoral, et, optionnellement, un dispositif destiné à recevoir la poudre d'hydroxyapatite et/ou de phosphate tricalcique et/ou un dispositif destiné à injecter *in vivo* la poudre ayant interagi avec un échantillon tumoral.

### DESCRIPTION DETAILLEE DE L'INVENTION

### Définitions

- Par "hydroxyapatite", on entend l'espèce minérale de la famille des phosphates, de formule Ca₅(PO₄)₃(OH), usuellement écrite Ca₁₀(PO₄)₆(OH)₂ pour souligner le fait que la maille de la structure cristalline comprend deux molécules. L'hydroxyapatite est le membre hydroxylé du groupe apatite. L'ion OH⁻ peut éventuellement être remplacé par le fluor, le chlore ou le carbonate.
- Par "frittage", on entend un procédé consistant à chauffer une poudre sans la mener jusqu'à la fusion. Ce procédé permet de maîtriser la densité de la poudre sans avoir de problème de la variation de volume et de dimensions de la poudre. Il permet également d'obtenir des matériaux durs. Le frittage peut être réalisé avec ou sans liant. Dans la présente invention, les termes "frittage" et "calcination" désignent la même chose.
- Par "granulométrie", on entend taille moyenne des particules, et plus précisément moyenne de la plus grande dimension des particules non sphériques ou diamètre pour des particules sphériques. Les méthodes de mesure de la granulométrie sont conventionnelles, par exemple par diffraction laser.

- Par "surface spécifique", on entend la superficie réelle de la surface d'un objet (ici un grain de la poudre d'hydroxyapatite ou de phosphate tricalcique) par opposition à sa surface apparente. La surface spécifique a une grande importance pour les phénomènes faisant intervenir les surfaces, comme l'adsorption, l'absorption ou les échanges de chaleur.
- Par "échantillon", on entend des cellules entières prélevées d'une tumeur, des broyats, des lyophilisats ou bien des dialysats de ces cellules ou un culot de centrifugation issues de ces cellules.
- Par "traitement", on entend une diminution d'une pathologie et/ou de ses symptômes, une stagnation de l'évolution de la pathologie et/ou de ses symptômes ou une récession complète de la pathologie et de ses symptômes et/ou une amélioration des conditions de vie. De manière générale, le terme "traitement" implique l'aspect curatif et préventif.
- Par "patient" ou "sujet", on entend un être humain ou un animal.
- Par "auto-vaccin", on entend un vaccin dans lequel les antigènes tumoraux proviennent d'une tumeur du patient destiné à être vacciné.
- Par "agent anti-tumoral", on entend un agent apte à faire régresser une tumeur tel qu'un agent hormonal et/ou un agent chimio-thérapeutique.
- Par "agent radio-thérapeutique", on entend une radiation ionisante utilisée localement pour détruire les cellules tumorales, cancéreuses. Les radiations ionisantes utilisées sont principalement les photons (ou rayons) X et gamma, les électrons, plus rarement les protons ou les neutrons. La "dose de rayonnement" correspond à la quantité d'énergie que les rayonnements/radiations vont déposer dans les tissus. Elle s'exprime en « grays » (Gy): un gray est égal à une énergie de un joule déposée dans un kilogramme de matière.

### Procédé de production d'une poudre d'hydroxyapatite et/ou de phosphate tricalcique

Le procédé de production d'une poudre d'hydroxyapatite et/ou de phosphate tricalcique décrit ici comprend au moins une étape de frittage d'une poudre d'hydroxyapatite et/ou de phosphate tricalcique à une température comprise entre 400°C et 600°C.

Il existe un grand nombre de procédés de production de l'hydroxyapatite phosphocalcique stœchiométrique Ca₁₀(PO4)₆(OH)₂. De préférence, le procédé utilisé ici consiste en une précipitation lente à haute température, obtenue par double décomposition d'un sel de calcium et d'un sel de phosphore en milieu basique. La réaction est longue, elle se passe à température constante dans un volume réactionnel important et est suivie d'une phase de maturation et d'une phase de lavage à l'eau.

Le précipité ainsi obtenu subit une nouvelle étape de transformation : la séparation solide/solution. Cette dernière étape est réalisée soit par filtration, séchage par étuvage et concassage, soit par atomisation à l'aide d'un lit fluidisé. L'atomisation permet d'avoir des particules sphériques qui lorsqu'elles sont utilisées dans une colonne de chromatographie ne se compactent pas et permettent le passage du fluide à moindre pression. Par ailleurs, ces particules sont plus faciles à injecter avec des aiguilles de petit diamètre.

Quelle que soit la technique de séparation solide/solution choisie, la poudre va subir deux étapes de transformations spécifiques à l'application de l'invention : l'étape de sélection granulométrique par tamisage à sec pour ne retenir que la tranche granulométrique d'intérêt, inférieure à 25µm ou comprise entre 25 et 45 µm, puis une dernière étape durant laquelle la poudre sera frittée à une température optimale pour assurer une fusion des grains conduisant à des états de surface des poudres bien particuliers, notamment caractérisés par des mesures de surface spécifique tels que définis dans la présente invention, à savoir ≥30 m²/g. Ces deux dernières étapes peuvent être inversées : sélection puis frittage ou frittage puis sélection.

Dans un mode de réalisation préféré, le frittage est obtenu par une montée en température effectuée avec un départ de la température ambiante pour atteindre une valeur d'environ 600°C, par exemple entre 500 et 800°C. Le retour à la température ambiante est réalisé, four fermé, selon un mode de descente libre.

En cours de procédé, des étapes de contrôle de la pureté de la poudre d'HAP peuvent être réalisés par diffraction des rayons X. La présence de phases étrangères (majoritairement de la chaux CaO et du phosphate tricalcique Ca₃(PO₄)₂) est recherchée. Des seuils d'acceptation de la présence de ces impuretés sont définis. Une caractéristique particulière de la poudre est mesurée, il s'agit de la largeur à mi-hauteur de certaines raies de diffraction du spectre de diffraction des rayons X de la poudre produite, afin de valider le parcours thermique de l'échantillon analysé.

### Paramètres de la synthèse (à titre d'exemple) :

- Sels en présence : la réaction chimique est réalisée préférentiellement en mettant en présence une solution de nitrate de calcium tetra hydraté avec une solution de phosphate d'ammonium (spontanément constituée lors du mélange de l'ammoniaque avec l'acide ortho phosphorique) en quantité convenable pour satisfaire à la stœchiométrie de la réaction chimique suivante. 10 Ca(NO₃)₂, 4H₂O + 6 H₃PO₄ + 20 NH₄OH → Ca₁₀(PO₄)₆(OH)₂ + 20 NH₄NO₃ + 22 H₂O
- L'alcalinisation du milieu est obtenue par ajout progressif de solution ammoniacale diluée.
- La température de réaction est maintenue à 70°C +/- 5°C à l'aide d'une double enveloppe dans laquelle circule un fluide caloporteur.
- Le volume réactionnel peut varier de 70 à 150 litres pour le mélange final.
- La durée de la réaction est de 3 à 5h00 en fonction du volume final souhaité.
- La sélection granulométrique est effectuée par tamisage à sec. La poudre retenue est inférieure à 25µm ou comprise entre 25 et 45µm.
- Le frittage est obtenu par une lente montée en température dans un four à moufle. Le rythme de montée en température du four ainsi que la valeur et la durée du plateau seront choisis afin d'obtenir des propriétés particulières de surface spécifique de l'hydroxyapatite, à savoir ≥30 m²/g. Préférentiellement, une montée en température est effectuée avec un départ de la température ambiante pour atteindre une valeur d'environ 600°C (500°C ; 800°C). Le retour à la température ambiante est réalisé, four fermé, selon un mode de descente libre.

### Paramètres du contrôle

- La pureté des hydroxyapatites obtenues est contrôlée par diffraction des rayons X, par comparaison aux données publiées dans un référentiel international : le fichier JCPDS (Joint Committee on Powder Diffraction Standards). Un pourcentage de ressemblance du spectre de la poudre obtenue avec les valeurs de diffraction d'une apatite standard répertoriée dans le référentiel JCPDS sous le numéro de fiche 09-0432 est recherché.
- Les impuretés attendues sont le phosphate tricalcique Ca₃(PO₄)₂ identifié en comparaison à la fiche JCPDS 09-0169 et/ ou la chaux CaO identifiée en comparaison à la fiche JCPDS 04-0777. Leur présence respective est tolérée à hauteur de 5 % environ pour chacune de ces phases.
- La mesure de la largeur à mi-hauteur de la raie majeure en intensité de diffraction de l'hydroxyapatite permet d'avoir des informations sur l'histoire thermique des poudres fabriquées.
- Sur un spectre de diffraction des rayons X, en se plaçant à une valeur d'angle en 2θ d'environ 31.773 degrés +/- 1%, ce qui correspond à la raie de diffraction majeure de l'hydroxyapatite, une mesure de la largeur à mi-hauteur est alors effectuée. La largeur à mi-hauteur à la hauteur totale de la raie à environ 31.773 degrés +/- 1% est alors reportée afin d'obtenir un pourcentage. Plus une hydroxyapatite est traitée thermiquement, plus elle est organisée d'un point de vue cristallographique. Plus les raies de diffraction des rayons X de son spectre sont fines et plus la largeur à mi-hauteur de la raie principale est faible. Cette mesure permet de qualifier l'histoire thermique de la poudre obtenue. Dans le procédé selon l'invention, un rapport de largeur à mi-hauteur sur la hauteur totale compris entre 0.2% et 0.35% et de préférence égal à 0.3% +/-10% est particulièrement souhaité.

### Poudre d'hydroxyapatite et/ou de phosphate tricalcique

La présente invention concerne une poudre d'hydroxyapatite et/ou de phosphate tricalcique obtenue(s) selon le procédé de production tel que défini ci-dessus.

La poudre d'HAP et/ou de phosphate tricalcique selon l'invention a des propriétés de surface autorisant, y compris sans agent couplant, la fixation spécifique des substances actives, et plus particulièrement d'antigènes tumoraux et leur transport vers des cellules du système des phagocytes mononucléés.

On choisit les poudres présentant au les caractéristiques suivantes :
- une granulométrie G telle que G ≤ 200µm, de préférence 0 < G ≤ 25µm ou comprise entre 25µm et 45µm, et
- une surface spécifique SS telle que SS ≥30 m²/g.

La poudre peut en outre présenter un rapport en pourcentage de la largeur à mi-hauteur sur la hauteur totale de la raie sur un spectre de diffraction des rayons X, en se plaçant à une valeur d'angle en 2θ d'environ 31.773 +/- 1%, compris entre 0,2% et 0,35%, de préférence égal à 0.3% +/-10%.

Dans un mode de réalisation encore plus préféré de l'invention, on choisit les poudres présentant, outre la granulométrie et la surface spécifique telles que définies ci-dessus, une forme de particules sphériques. Les particules de forme sphériques sont particulièrement adaptées à la mise en œuvre selon l'invention, contrairement aux particules en forme d'aiguille.

La granulométrie de poudre est choisie de sorte à ce que la poudre puisse pénétrer dans les cellules à cibler. Par ailleurs, la quantité de protéines fixées sur la poudre varie en fonction de la surface spécifique de la poudre. Ce paramètre a donc des conséquences directes sur les propriétés finales de la poudre pour induire une réponse immunitaire et engendrer un traitement des cellules ou de la pathologie visée. Enfin, la forme sphérique des particules permet une meilleure fixation des protéines spécifiques mais surtout une meilleure résistance physique des particules de poudre dans la colonne, en particulier lorsque celle-ci est mise sous pression manuellement. Il est donc important de trouver un juste équilibre entre ces paramètres pour avoir la poudre et une composition à visée thérapeutique optimale.

La granulométrie et la surface spécifique sont influencées par la température de frittage mise en œuvre dans le procédé de production de la poudre d'HAP et/ou de phosphate tricalcique. En effet, lorsque la température de frittage est trop élevée dans le procédé de production de la poudre d'HAP et/ou de phosphate tricalcique mentionné ci-dessus, la poudre d'HAP et/ou de phosphate tricalcique obtenue n'a pas une surface spécifique satisfaisante car elle ne permet pas de fixer suffisamment de protéines et, lors de son introduction *in vivo* chez le patient, elle ne va donc pas induire une réaction immunitaire suffisante via les cellules présentatrices d'antigènes. En revanche, si la température de frittage est trop basse ou nulle dans le procédé de production de la poudre d'HAP et/ou de phosphate tricalcique, la poudre obtenue n'est pas assez résistante et elle devient soluble aux différents pH qui se trouvent dans l'environnement cellulaire. De plus, une fois humides, les poudres non sphériques, non céramisées et avec une granulométrie inadaptées ont tendance à s'agglomérer. Il est donc nécessaire qu'elles ne soient pas trop sensibles à l'humidification. Ainsi, avec une température de frittage trop basse, les colonnes faites de poudre d'HAP et/ou de phosphate tricalcique, sur lesquelles va percoler un échantillon tumoral en vue de retenir les protéines spécifiques de la tumeur du patient (et en particulier les protéines du choc thermique), vont colmater et ne plus laisser passer aucun liquide. De plus, les poudres très solubles relarguent beaucoup de calcium et peuvent, en plus du désavantage de colmater, faire polymériser la fibrine contenue généralement dans les échantillons à percoler et ainsi obstruer la colonne.

Il est du mérite des inventeurs d'avoir mis en évidence que la température optimale de frittage pour la préparation de la poudre d'HAP et/ou de phosphate tricalcique est comprise entre 400 et 600 °C.

La poudre d'HAP et/ou de phosphate tricalcique obtenue via le procédé de production ci-dessuspeut alors être utilisée directement, une fois chargée en protéines spécifiques d'une tumeur d'un patient, en tant que médicament injectable directement dans l'organisme en vue de traiter ladite tumeur.

Ce nouveau procédé de production de poudre d'hydroxyapatite et/ou de phosphate tricalcique permet de disposer d'une nouvelle poudre d'HAP et/ou de phosphate tricalcique qui est un nouveau matériau vecteur plus performant effectuant à la fois, la purification, la vectorisation dans l'organisme et le transport des substances actives dans les cellules cibles : les cellules présentatrices d'antigènes (APCs).

### Procédé de préparation d'une composition pharmaceutique

La présente invention concerne une composition à base de ladite poudre d'HAP et/ou de phosphate tricalcique.

Le procédé de préparation de la composition pharmaceutique est caractérisé en ce qu'il comprend les étapes suivantes :
a) la préparation d'un échantillon tumoral,
b) l'extraction des protéines cytoplasmiques de l'échantillon tumoral,
c) le passage des protéines extraites à l'étape b) au travers d'une colonne de chromatographie contenant une poudre d'hydroxyapatite et/ou de phosphate tricalcique telle que définie ci-dessus,
d) éventuellement au moins un lavage de la poudre à l'issue de l'étape c).

La préparation de l'échantillon tumoral (étape a) consiste au prélèvement d'un échantillon cellulaire in vivo, directement dans la tumeur du patient. De préférence, le prélèvement se fait par ponction dans la tumeur, par exemple à partir d'une biopsie d'un patient atteint de cancer. Il peut également s'agir d'échantillons tumoraux comprenant des cellules tumorales du patient à traiter, déjà prélevés préalablement, ces derniers étant des broyats, des lyophilisats, des dialysats ou un culot de centrifugation.

L'extraction des protéines cytoplasmiques de l'échantillon tumoral (étape b) peut se faire selon des méthodes classiques connues. Dans un mode de réalisation, la méthode d'extraction vise à extraire l'ensemble des protéines cytoplasmiques des cellules tumorales de l'échantillon, en particulier elle ne comprend pas d'étape de sélection ou de purification de protéines spécifiques et notamment d'antigènes tumoraux éventuellement contenus dans les protéines cytoplasmiques.

De préférence, l'étape d'extraction des protéines cytoplasmiques de l'échantillon tumoral est avantageusement effectuée de la façon suivante :
1) éventuellement congélation du tissu tumoral,
2) broyage du tissu tumoral,
3) solubilisation ou mise en suspension des antigènes tumoraux cytoplasmiques dans une solution de NaHCO3,
4) centrifugation,
5) séparation du culot et du surnageant.

L'extrait de protéines cytoplasmiques est mis à passer ou percoler au travers d'une colonne de chromatographie contenant la poudre d'HAP et/ou de phosphate tricalcique selon l'invention et telle que définie ci-dessus (étape c du procédé de préparation de la composition). Cette étape consiste à mettre en contact de la poudre d'HAP et/ou de phosphate tricalcique selon l'invention avec l'extrait protéique issu de l'étape b du procédé. La poudre d'HAP et/ou de phosphate tricalcique selon l'invention a l'avantage d'être suffisamment résistante pour ne pas colmater dans la colonne et pouvoir ainsi laisser "passer" l'extrait protéique de l'échantillon tumoral qui va percoler dans la colonne.

Avantageusement, la colonne de chromatographie contenant la poudre d'HAP et/ou de phosphate tricalcique selon l'invention est mise sous pression. La solution de protéines qui va percoler sur la colonne contenant la poudre d'HAP et/ou de phosphate tricalcique ne peut théoriquement pas passer au travers de la poudre à pressions atmosphérique, c'est pourquoi il est nécessaire, dans ce cas, d'utiliser une colonne pouvant être mise sous pression manuellement via un piston par exemple. En pratique, la mise en contact peut être, par exemple, une purification réalisée à l'aide d'une ou plusieurs colonne séparées ou pas par un système de réservoir dans lequel peuvent être introduites ou soustraites certaines solutions et au travers de laquelle ou desquelles on peut faire percoler un broyat de tumeur. Dans un mode de réalisation particulier, si nécessaire, on peut récupérer la (ou les) protéines adsorbées sur la colonne de chromatographie par exemple, par élution de la (ou des) colonne(s) au moyen d'une solution de tampon - de préférence de tampon phosphate - de molarité et de pH appropriés, l'éluat ainsi obtenu comprenant des antigènes tumoraux et/ou les facteurs adjuvants immobilisés et recherchés. Il s'agit ici d'une technique de chromatographie.

Dans un autre mode de réalisation préféré, on récupère une composition comprenant la (ou des) protéine(s) de la tumeur d'un patient adsorbées sur la poudre d'HAP et/ou de phosphate tricalcique.

L'étape de lavage de la colonne (étape d) permet de séparer la poudre d'HAP et/ou de phosphate tricalcique (support minéral) des protéines de l'échantillon tumoral non spécifiquement adsorbées. Cette étape est avantageusement effectuée à plusieurs reprises avec des tampons phosphate ou de solution saline de concentration croissante. Ainsi, le premier lavage est effectué avec un tampon phosphate ou une solution de NaCl de concentration inférieure ou égale à 200mM alors qu'un deuxième lavage peut être effectué avec un tampon phosphate ou une solution de NaCl de concentration comprise entre 300 à 500 mM.

Ainsi, il a été constaté par les inventeurs que la poudre d'HAP et/ou de phosphate tricalcique selon l'invention, après avoir été mise en contact avec les protéines extraites de l'échantillon tumoral fixent avantageusement les protéines spécifiques d'une tumeur d'un patient donné ou les protéines permettant une stimulation du système immunitaire, qui représentent les protéines immunogènes.

Dans un mode de réalisation particulier de l'invention, une fois adsorbée, la poudre d'HAP et/ou de phosphate tricalcique issue de l'étape c) ou d) du procédé de préparation de la composition pharmaceutique est avantageusement remise en suspension avec une solution adaptée, de préférence un liquide d'injection (étape e) pour pouvoir être ensuite administrée au patient. On peut avantageusement y ajouter de la CarboxyMethyl Cellulose (CMC), un excipient permettant de faciliter l'injection. C'est ainsi que la poudre d'HAP et/ou de phosphate tricalcique portant les protéines spécifiques de la tumeur d'un patient est utilisée directement en tant que composition pharmaceutique ou médicament.

Dans une forme préférée de l'invention, la colonne de chromatographie contenant la poudre d'HAP et/ou de phosphate tricalcique selon l'invention a la capacité d'être fermée à ses deux extrémités. Ceci permet d'injecter un liquide de mise en suspension ou une solution ou liquide d'injection dans la colonne et de pouvoir ensuite administrer directement au sujet ou patient. L'obturation de la colonne aux deux extrémités est avantageuse car, une fois le liquide d'injection introduit dans la colonne, cette dernière est agitée afin de mettre correctement en suspension la poudre selon l'invention chargée de protéines, de préférence de protéines spécifiques de la tumeur du patient. La non-ouverture de la colonne est un avantage considérable car il permet de limiter les risques de contamination.

Dans cette forme de réalisation particulière, le procédé de préparation du médicament comprend la succession des étapes suivantes :
a) la préparation de l'échantillon tumoral comprenant des cellules tumorales, par exemple du sujet à traiter,
b) l'extraction des protéines cytoplasmiques de l'échantillon tumoral permettant la mise en suspension ou la solubilisation des antigènes tumoraux d'intérêt,
c) la percolation desdites protéines cytoplasmiques de l'échantillon tumoral au travers d'au moins une colonne de chromatographie contenant la poudre d'HAP et/ou de phosphate tricalcique selon l'invention,
d) éventuellement le(s) lavage(s) de la colonne de chromatographie par des solutions tampon de force ionique et pH déterminés,
d-bis) la fermeture de la colonne de chromatographie contenant la poudre d'HAP et/ou de phosphate tricalcique selon l'invention,
e) la mise en suspension de la poudre d'HAP et/ou de phosphate tricalcique dans une solution d'injection.

L'étape d-bis) se produit qu'il y ait ou non l'étape concernant le(s) lavage(s) de la colonne de chromatographie.

### Composition pharmaceutique

La composition selon l'invention est issue du procédé de production tel que défini ci-dessus. Elle correspond à la poudre d'HAP et/ou de phosphate tricalcique selon l'invention chargée des protéines adsorbées sur ladite poudre d'HAP et/ou de phosphate tricalcique, selon le procédé de l'invention.

Sans être lié par une quelconque théorie, la composition selon l'invention ainsi obtenu comprend des antigènes tumoraux associés de manière non covalente à la poudre d'HAP et/ou de phosphate tricalcique.

En particulier, dans un mode de réalisation préférée, la composition selon l'invention comprend des antigènes tumoraux associés de manière non covalente à la poudre d'HAP et/ou de phosphate tricalcique, choisis parmi :
- les protéines qui se fixent au CD91.
- les protéines du choc thermique ; HSP70, gp96, HSP27 et leurs peptides associés.
- β-catenine, P-cadherine, Her-2/neu.

De préférence, au moins 90%, par exemple au moins 95%, voire au moins 99% des protéines (mole%) se fixant sur la membrane cellulaire des APCs se fixent aux récepteurs CD91. Les protéines vaccinales se fixant sur d'autres récepteurs cellulaires n'ont pas été détectées par immunohistochimie.

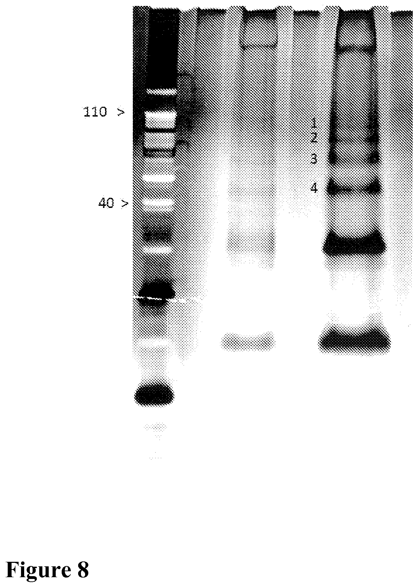

Afin de caractériser la composition telle qu'obtenue après adsorption de protéines de tumeurs, les inventeurs ont constaté qu'après migration sur gel d'électrophorèse, les compositions selon l'invention comprennent toutes au moins quatre bandes de migration de poids moléculaires compris entre 110 et 40 kDa. (voir **Figure 8**).

Dans une variante de réalisation, la composition selon l'invention est issue du procédé de préparation comprenant les étapes suivantes :
a. la préparation d'un échantillon tumoral comprenant des cellules tumorales, par exemple du sujet à traiter,
b. l'extraction des protéines cytoplasmiques de l'échantillon tumoral,
c. le passage des protéines extraites à l'étape b) au travers d'une colonne de chromatographie contenant une poudre d'hydroxyapatite et/ou de phosphate tricalcique ayant une surface spécifiques SS telle que SS ≥ 30 m²/g,
d. éventuellement au moins un lavage de la poudre à l'issue de l'étape c.

La composition selon l'invention peut comprendre en outre des excipients ou véhicules pharmaceutiques appropriés. Dans un mode de réalisation particulier, la composition selon l'invention comprend du carboxyméthylcellulose, utilisé comme additif pour maintenir les poudres en suspension dans la solution. Par exemple, il s'agit d'une solution d'injection constituée de carboxymethyl cellulose à 2% dans une solution de NaCl 0,02M.

### Utilisation de la composition

La composition selon l'invention telle que décrite ci-dessus peut être avantageusement utilisée comme médicament.

Elle intervient par exemple dans le traitement des tumeurs.

La composition selon l'invention est utilisée dans le traitement du lymphome B ou T.

Cette composition est utile notamment dans le traitement de pathologies chez l'homme, ou chez l'animal, par exemple le mammifère, et notamment le chien, le cheval ou le chat. Il a été montré en particulier que l'utilisation de la composition donne des résultats particulièrement probants et intéressants dans le traitement des pathologies canines, et par exemple dans le traitement de l'ostéosarcome du chien et les lymphomes B ou T du chien ou dans le traitement de pathologies équines comme le mélanome du cheval ou encore dans le traitement de pathologies félins comme le fibrosarcome du chat (cf. exemples plus bas). Cependant, ces traitements peuvent être appliqués à tout être vivant, humain, mammifères ou autres animaux susceptibles de développer des tumeurs cancéreuses.

La composition selon l'invention comprend donc la poudre d'HAP et/ou de phosphate tricalcique ayant adsorbée les antigènes tumoraux spécifiques du patient et ayant été remise en suspension. La composition peut comprendre de préférence les antigènes suivants : les protéines du choc thermique, HSP70, gp96, HSP27 et leurs peptides associés, β-caténine, P-cadhérine, Her-2/neu. De préférence, au moins 90%, par exemple au moins 95%, voire au moins 99% des protéines (mole%) se fixant sur la membrane cellulaire des APCs se fixent aux récepteurs CD91. Elle est utilisée par exemple pour des traitements autologues par immunothérapie, en particulier comme vaccin anti-tumoral. Il s'agit d'une composition utilisée comme auto-vaccin, de préférence comme auto-vaccin anti-tumoral.

La composition est susceptible d'être obtenue par le procédé comprenant les étapes suivantes :
a. la préparation d'un échantillon tumoral comprenant des cellules tumorales, par exemple du sujet à traiter,
b. l'extraction des protéines cytoplasmiques de l'échantillon tumoral,
c. le passage des protéines extraites à l'étape b) au travers d'une colonne de chromatographie contenant une poudre d'hydroxyapatite et/ou de phosphate tricalcique,
d. éventuellement au moins un lavage de la poudre à l'issue de l'étape c),

Selon la présente divulgation, cette composition peut-être utilisée dans le traitement des pathologies suivantes ou d'au moins l'une des pathologies suivantes :
les ostéosarcomes, les lymphomes B ou T, les tumeurs mammaires, les mélanomes, les hémangio sarcomes, les mastocytomes, les fibrosarcomes, les tumeurs cérébrales ou du système nerveux central, les schwanomes, les mésothéliomes, les séminomes, les tératomes, ou les blastomes chez un sujet, homme ou animal.

La composition en question comprend donc une poudre d'hydroxyapatite et/ou de phosphate tricalcique ayant subi une étape de frittage limitée à une température donnée, telle que comprise entre 400°C et 600°C.

Dans une autre forme particulière de réalisation, la composition selon l'invention peut être utilisée pour une utilisation thérapeutique en association avec un agent thérapeutique, de préférence un agent anti-tumoral et/ou un agent radio-thérapeutique.

Tant qu'un état d'aplasie ou de quasi-aplasie n'est pas atteint chez le sujet à traiter, il est possible d'associer avantageusement l'immunothérapie (liée à la poudre selon l'invention) et la chiomiothérapie. L'effet des molécules de chimiothérapie sur les lymphocytes T régulateurs permet sans doute de rééquilibrer la balance des cellules immunitaires et des possibilités d'immunisation. Ainsi, l'immunothérapie peut être associée à tout type d'anticancéreux tant que la lymphopénie n'est pas trop importante. Elle peut être associée aux anticancéreux à dose cytotoxiques ou à dose plus faibles dites métronomiques, par exemple à des doses de cyclophosphamide de 50 mg/jours associées à 2,5 mg de methotrexate au premier et second jour de traitement, dans le cas de cancer du sein récurrents chez l'humain.

Comme agent anti-tumoral, il peut s'agir de molécules chimiothérapeutiques. Ces dernières sont classées en agent alkylants, antimétabolites, alcaloïdes végétaux, inhibiteurs de la topoisomérase et antibiotiques antitumoraux. Tous ces médicaments affectent à un certain niveau la mitose et/ou la synthèse et la fonction de l'ADN.

Il peut s'agir également d'agents n'agissant pas directement sur l'ADN comme des inhibiteurs de la tyrosine kinase et en particulier de l'inhibiteur de la tyrosine kinase mésylate d'imatinib qui cible directement une anomalie moléculaire dans certains types de cancers tels que la leucémie et le cancer du côlon.

D'autres médicaments d'association à la poudre selon l'invention modifient le comportement des cellules tumorales sans pour autant attaquer directement les cellules. On utilise notamment les hormones pour ce genre de thérapie adjuvante.

Les agents alkylants ont la capacité d'ajouter un groupe alkyle à un grand nombre de groupes électronégatifs dans la cellule. Ils limitent la croissance cellulaire en liant les nucléotides guanines de la double hélice d'ADN. Les deux brins d'ADN ne peuvent ainsi plus se dérouler, ni se séparer. La cellule ne peut alors plus se diviser. Ces agents n'agissent généralement pas spécifiquement sur la cellule cancéreuse et certains nécessitent une conversion in vivo en substances actives (par exemple le cyclophosphamide). Comme exemple d'agent alkylants utilisables en chimiothérapie, on peut citer : cisplatine, carboplatine (ou paraplatine), ifosfamide, chlorambucil, busulfan, thiotépa. Parmi les agents alkylants, le cyclophosphamide a montré être particulièrement bénéfique pour l'immunothérapie. Cette molécule potentialise les réactions d'hypersensibilité retardée. Le mécanisme de potentialisation de l'hypersensibilité retardée se fait par la réduction des fonctions des lymphocytes T régulateur.

Les antimétabolites agissent en prenant la place des purines ou des pyrimidines qui sont des nucléotides, composants élémentaires de l'ADN. Des erreurs dans l'agencement des bases sont ainsi faîtes lors de la réplication de l'ADN pendant la phase S du cycle cellulaire, arrêtant ainsi le développement et la division cellulaire et à une apoptose.

Les antimétabolites se répartissent en plusieurs groupes selon le type de cible qu'ils atteignent :
- les antipyrimidines. Parmi ceux-ci, on peut citer le 5-fluorouracile (5-FU) qui inhibe la thymidylate synthase.
- les antipurines. Parmi ceux-ci, on peut citer la fludarabine qui inhibe l'ADN polymérase, l'ADN primase et l'ADN ligase I et est exclusivement active lors de la phase S étant donné que ces enzymes sont très actives lors de la réplication cellulaire.
- les antifolates. Le méthotrexate (antagoniste du folate) inhibe la dihydrofolate réductase, une enzyme essentielle à la synthèse des purines et des pyrimidines.
- l'hydroxyurée.

Les alcaloïdes sont des dérivés des végétaux et bloquent la division cellulaire en empêchant la synthèse des microtubules et la formation du fuseau mitotique, vital pour la division cellulaire.

Parmi les alcaloïdes utilisables dans la présente composition, on peut citer :
- les vinca-alcaloïdes comme la vincristine, la vinblastine ou la vinorelbine qui se lient à des sites spécifiques de la tubuline et inhibent l'assemblage des tubulines en microtubules, essentiels à la division cellulaire,
- les taxanes tels que paclitaxel (de Taxus brevifolia) avec son dérivé synthétique docétaxel) qui inhibe la division cellulaire en stimulant la polymérisation des tubulines, améliorant la formation et la stabilité des microtubules qui ne peuvent alors pas se dégrader, empêchant ainsi les chromosomes de migrer vers les pôles du noyau,
- les épithilones, produits d'une myxobactérie qui ont le même mécanisme que les taxanes et semblent avoir une activité anticancéreuse similaire.

Les inhibiteurs des topoisomérases agissent par définition sur les topoisomérases qui sont des enzymes essentielles maintenant la topologie de l'ADN. L'inhibition de la topoisomérase de type I ou de type II gênent à la fois la transcription et la réplication de l'ADN en dérangeant le superenroulement de l'ADN.

Comme inhibiteurs des topoisomérase de type I, on peut citer les dérives de la camptothécine.

Comme inhibiteurs des topoisomérase de type II, on peut citer l'amsacrine, les anthracyclines, les dérives de l'épipodophyllotoxine.

Les antibiotiques antitumoraux sont nombreux et de types différents. Généralement, ils empêchent la division cellulaire par plusieurs moyens :
- la liaison à l'ADN, en s'intercalant entre deux bases de nucléotides adjacents et en les empêchant de se séparer,
- l'inhibition de l'ARN, empêchant la synthèse d'enzymes,
- la gêne de la réplication cellulaire.

Ils sont produits par diverses souches de la bactérie Streptomyces.

Parmi ceux-ci, on peut citer les anthracyclines : doxorubicines et daunorubicine qui inhibent aussi la topoisomérase de type II, l'actinomycine D, la mitomycine C, la plicamycine, la bléomycine qui agit de manière unique en oxydant le complexe ADN-bléomycine-Fe(II) formant ainsi des radicaux libres qui induisent des dommages et des aberrations chromosomiques. Parmi ceux- ci on peut classer les antiasparagines (L-asparaginase). L'asparagine est un acide aminé essentiel pour certaines cellules cancéreuses qu'elles sont incapables de synthétiser au contraire des cellules normales. Elles dépendent donc de l'asparagine circulante.

Il existe également une nouvelle classe de médicaments anticancéreux dont l'association à un intérêt avec les vaccins antitumoraux : les anticorps monoclonaux tels que les anti VEGF, anti CD20, CD19, CD30 et autres.

On a découvert les conséquences biologiques des radiations ionisantes sur les tissus vivants peu après la découverte des rayons X. La plupart des données radiobiologiques montrent que l'ADN est la cible la plus importante pour les effets biologiques sous forme de liaison des bases, de cassure de brin au niveau de la liaison sucre-phosphate. Dans la cellule la lésion radio induite entraîne des mutations, et/ou une impossibilité de la division cellulaire. Ces conséquences sont plus importantes pour les cellules en prolifération comme les cellules tumorales mai également les cellules saines entraînant des effets secondaires également importants.

On peut distinguer quatre grandes techniques de radiothérapie : la radiothérapie externe, la radiochirurgie, la curiethérapie et la radiothérapie métabolique. Chacune d'elle possède ses indications selon le type de tumeur et sa localisation.
- En radiothérapie externe, la plus utilisée, la source de rayonnement est à l'extérieur du sujet à traiter. Les bombes au cobalt, qui utilisent une source radioactive y de cobalt 60, ont pratiquement disparu au profit des accélérateurs linéaires d'électrons produisant des faisceaux de rayons X haute énergie et des faisceaux d'électrons. Il existe trois techniques principales : la radiothérapie conventionnelle, la radiothérapie conformationnelle 3D (en 3 dimensions) et la tomothérapie ou radiothérapie hélicoïdale.
- En curiethérapie, la source radioactive est placée pendant une durée limitée (le plus souvent quelques heures) ou définitivement, à l'intérieur du sujet à traitre, dans la tumeur ou dans une cavité à son contact. Trois techniques principales, elles-mêmes se subdivisent en sous-techniques suivant leur débit de dose (bas débit et haut débit) et leur type de chargement (manuel ou différé). Il s'agit de la curiethérapie interstitielle, la curiethérapie endocavitaire et la curiethérapie endoluminale.
- En radiothérapie métabolique, la source radioactive non scellée, sous forme liquide ou de gélule, est injectable et va se fixer sur les cellules cibles.

En fonction du type de la tumeur, de sa localisation, de sa taille, de son extension et de son stade, de l'état général du sujet à traiter et des symptômes associés, on distingue trois situations très différentes dans lesquelles on va utiliser la radiothérapie dans des buts bien précis :
- La radiothérapie curative dont l'objectif est d'irradier la plupart des cellules cancéreuses afin d'entraîner le contrôle voire la guérison du cancer. Cela implique l'absence de lésions à distance. Elle est indiquée dans environ la moitié des irradiations. Elle est avantageusement utilisée seule ou en association avec la chirurgie et/ou la chimiothérapie. La dose nécessaire dépend du type et du volume de la tumeur, certaines étant très radiosensibles alors que d'autres sont radiorésistantes. Le protocole habituel délivre une dose de 10 Gy par semaine à raison de 5 séances de 2 Gy par jour. La dose totale varie selon les cas de 30 à 70 Gy.
- La radiothérapie palliative dont l'objectif n'est pas ici de guérir le cancer mais de soulager le sujet à traiter par de légères doses, permettant d'atténuer la douleur résultant de cancer trop avancé pour être soigné. Elle s'adresse aux cancers trop évolués localement ou métastatiques. Le traitement étant palliatif, il doit être de courte durée et peu agressif, pour entraîner le moins de désagréments possible au patient.
- La radiothérapie symptomatique dont l'objectif est de soulager un symptôme majeur particulièrement gênant pour le malade. Son efficacité est : antalgique, hémostatique, ou décompressive.

Dans toutes ces techniques, les radiations utilisées sont les photons (ou rayons) X et gamma, les électrons, plus rarement les protons ou les neutrons.

L'invention concerne donc également une combinaison de traitement thérapeutique comprenant l'administration d'une dose efficace de la poudre d'hydroxyapatite et/ou de phosphate tricalcique telle que définie ci-dessus et/ou de la composition de l'invention telle que définie ci-dessus avec au moins une dose efficace d'un agent thérapeutique, de préférence un agent anti-tumoral et/ou un agent radio-thérapeutique tels que définis ci-dessus.

L'administration de la composition selon l'invention (auto-vaccin antitumoral) et de l'agent anti-tumoral peut se faire concomittamment, sous la forme d'une seule composition ou de deux compositions séparées, ou à des temps différents, selon le protocole optimal selon le patient et la pathologie à traiter.

Les inventeurs ont montré que, de manière remarquable, une rémission complète de certaines tumeurs, notamment chez le chien lorsque la composition selon l'invention est utilisée en association avec un autre agent anti-tumoral (cf. exemples plus bas).

La composition telle que définie ci-dessus combinée avec au moins un agent thérapeutique tel qu'un agent anti-tumoral défini ci-dessus et/ou au moins un agent radio-thérapeutique représente donc également une association médicamenteuse couverte par la présente invention.

La composition selon l'invention est administrée de préférence par injection.

Lorsqu'il s'agit de l'association médicamenteuse, l'injection de la composition et de l'agent thérapeutique peut être simultanée ou indépendante dans le temps.

L'un des avantages de la composition selon l'invention est qu'elle est particulièrement fiable sur le plan de la contamination. En effet, son procédé de production peut se faire quasiment en milieu totalement fermé et avec la même poudre d'HAP et/ou de phosphate tricalcique, ce qui limite les risques de contamination. Il n'y a, de préférence, pas de transfert d'un support d'HAP et/ou de phosphate tricalcique à un autre et c'est la poudre d'HAP et/ou de phosphate tricalcique ayant servi à la purification et à la fixation des matériaux biologiques (de préférence les protéines spécifiques de la tumeur donnée du patient et plus particulièrement les protéines du choc thermique) qui sera injectée directement sans être prélevée auparavant. La composition pharmaceutique est ainsi constituée à la fois par la poudre d'HAP et/ou de phosphate tricalcique mais également par les matériaux biologiques qui sont adsorbées sur cette dernière.

Une autre caractéristique avantageuse de la composition tient à ce qu'elle est destinée à être phagocytée par des macrophages et/ou d'autres cellules APC et/ou des cellules dendritiques. Elle permet également le transport in vivo d'une ou plusieurs substances actives. En d'autres termes, la poudre d'HAP et/ou de phosphate tricalcique selon l'invention permet de vectoriser toute substance/molécule adsorbable sur cette dernière, en utilisant les cellules présentatrices de l'antigène APCs ou autres cellules analogues.

La composition peut être destinée à activer des macrophages et/ou d'autres cellules APC, c'est-à-dire entraîner la synthèse de substances choisies dans le groupe comprenant les cytokines, les lymphokines, les facteurs de croissance et/ou rendre les cellules dendritiques matures.

Une fois injectée dans un tissu conjonctif, la composition selon l'invention peut entraîner un afflux local de macrophages, de cellules dendritiques et/ou d'autres cellules APC présentatrices de l'antigène ou des antigènes adsorbé(s) sur la poudre d'HAP et/ou de phosphate tricalcique.

Suivant une modalité intéressante, la composition selon l'invention, une fois administrée *in vivo* ou *in vitro*, permet la libération prolongée (par exemple pendant quelques heures à plusieurs jours) de la (ou des) substances active(s) adsorbée(s) sur la poudre d'HAP et/ou de phosphate tricalcique.

Lorsqu'elle est employée sous la forme d'une unité galénique, la composition pharmaceutique ou médicament selon l'invention est avantageusement employée en quantité comprise entre 15 et 100 µg dose/unité galénique.

Dans un mode de réalisation particulier, une dose administrée par injection chez le patient, comprend entre 30 et 50 mg d'hydroxyapatite et entre 1000 et 2000 µg de protéines, de préférence 100% ou, au moins 90%, par exemple au moins 95%, voire au moins 99% des protéines (mole%) se fixant sur la membrane cellulaire des APCs se fixent aux récepteurs CD91. Au sens de l'invention, le terme CD91 fait référence au récepteur transmembranaire exprimé chez l'homme, en particulier de séquence protéique (UNIPROT) Q07954, aussi appelé LRP1, et interagissant avec les protéines HSP, notamment gp96.

Dans une forme particulière d'utilisation de la composition, il est avantageux d'effectuer une revaccination des sujets atteints. Ainsi, dans un mode de réalisation spécifique, il est préférable de procéder à deux étapes successives d'administration de l'auto-vaccin anti-tumoral. En particulier, le deuxième vaccin est de préférence obtenu à partir d'un nouveau prélèvement de tumeurs chez le sujet atteint.

En effet, il est supposé que les traitements anti-tumoraux éliminent les différents clones constitutifs d'une tumeur de manière différentielle en raison de leur sensibilité variable aux traitements. Les clones les plus sensibles seraient éliminés et les plus résistants se développeraient pour occuper la niche laissée par les précédents. C'est pourquoi, lors d'un échappement de la tumeur à la première réponse vaccinale, il est justifié de procéder à un second vaccin à partir d'un nouveau prélèvement afin de stimuler le système immunitaire contre l'ensemble des protéines anormales caractérisant les nouveaux clone dominants.

### Kit de mise en œuvre du procédé de préparation de la composition

La divulgation vise en outre un kit pour la mise en œuvre du procédé de préparation de la composition tel que défini ci-dessus (avec ou sans étape de frittage de la poudre), caractérisé en ce qu'il comprend la poudre d'hydroxyapatite et/ou de phosphate tricalcique telle que définie ci-dessus, destinée à interagir avec les protéines cytoplasmiques extraite d'un échantillon tumoral.

Ce kit comprend optionnellement un dispositif destiné à recevoir la poudre d'hydroxyapatite et/ou de phosphate tricalcique et/ou un dispositif destiné à administrer, de préférence à injecter, *in vivo* ladite poudre ayant interagi avec les protéines cytoplasmiques d'un échantillon tumoral. Le dispositif destiné à administrer *in vivo* la poudre peut être par exemple une seringue.

Ce kit permet avantageusement à un praticien en médecine animale ou humaine de purifier les peptides spécifiques de la tumeur grâce à des poudres d'HAP et/ou de phosphate tricalcique, de les fixer sur ces mêmes poudres qui seront ensuite injectées chez un patient. De préférence, les peptides spécifiques de la tumeur liés aux protéines du choc thermique fixés à la poudre d'HAP et/ou de phosphate tricalcique proviennent du patient chez qui la poudre sera ré-administrée, de préférence réinjectée. L'injection chez le patient est réalisée de préférence dans les tissus en sous-cutanée ou en intradermique.

Dans une variante de réalisation du kit, ce dernier comprend éventuellement un ou plusieurs des moyens suivants, outre la poudre d'hydroxyapatite et/ou de phosphate tricalcique:
- les moyens de prélèvement de l'échantillon tumoral
- les moyens d'extraction des protéines cytoplasmiques de l'échantillon tumoral, tels qu'une solution de NaHCO₃ ou Na₂CO₃.
- les moyens permettant de fixer les protéines spécifiques de la tumeur à la poudre d'hydroxyapatite et/ou de phosphate tricalcique,
- les moyens permettant la mise en suspension de la poudre d'HAP et/ou de phosphate tricalcique chargée en protéines spécifiques de la tumeur,
- les moyens permettant d'administrer, de préférence injecter, *in vivo* ladite suspension.

Il ressort notamment de l'exposé ci-dessus de l'invention que les particules d'HAP et/ou de phosphate tricalcique selon l'invention sont un bon vecteur pour transporter les protéines spécifiques de la tumeur d'un patient, à l'intérieur des macrophages et des cellules qui présentent les antigènes et qu'elles sont adaptées pour traiter des pathologies telles que le cancer. Ceci sera plus clairement vu à la lecture des exemples suivants.

### DESCRIPTION DES FIGURES

**Figure 1****:** La **figure 1** illustre le spectre de diffraction des rayons X d'une poudre d'hydroxyapatite frittée à 600°C selon l'invention. En abscisses figure 2 téta correspondant à l'angle de diffraction et en ordonnées l'intensité (hauteur) des différents pics.
**Figure 2** **:** La **figure 2** illustre la mesure de l'activité phosphatase alcaline après 1^{1/2}h de mise en contact de la poudre avec des cellules et ceci en fonction des différentes doses de poudre.
**Figure 3** **:** La **figure 3** illustre la mesure de l'activité phosphatase alcaline après 3^{1/2}h de mise en contact de la poudre avec des cellules et ceci en fonction des différentes doses de poudre.
**Figure 4** **:** La **figure 4** illustre la mesure de l'activité phosphatase alcaline après 7^{1/2}h de mise en contact de la poudre avec des cellules et ceci en fonction des différentes doses de poudre.
**Figure 5** **:** ▲ : aucun traitement ; ■ : vaccin selon l'invention ; ◆: chimiothérapie selon Takuo Shida et coll. Low-dose chemotherapy for canine appendicular osteosarcoma, Journal of Japan Veterinary Cancer Society, vol.2, No.1, 1-6 2011
**Figure 6** **:** La **figure 6** illustre les résultats de l'étude. Il s'agit de trois courbes montrant la survie en jours et le pourcentage de survie des chiens atteints de lymphome B traités par l'auto-vaccin selon présente invention seul (-▲-), des chiens traités par l'auto-vaccin selon présente invention en association avec une chimiothérapie (-◆-) et des chiens traités par un placebo en association avec une chimiothérapie (-■-).
**Figure 7** **:** protocoles chimiothérapie + vaccins :
   Asp correspond à L-Asparaginase à 400UI/kg.
   Vinc correspond à la vincristine à 0.75mg/m².
   Cyclo correspond à la cyclophosphamide à 250 mg/m².
   Adri correspond à l'adriblastine en IntraVeineuse (IV) à 30mg/m².
   Lomu correspond à la lomustine à 60-80mg/m².
   Case grise correspond à la prednisone à 1mg/kg/jour.
   Case noire correspond à la prednisone par voie orale à 1mg/kg/jour/2.
   X correspond à une injection du vaccin comprenant la composition selon l'invention.
**Figure 8** **:** Gel d'électrophorèse des protéines des compositions selon la présente invention montrant la présence de 4 bandes de migration entre 40 et 110 kDa.

### EXEMPLES

### Exemple 1 : Procédé de préparation de la poudre d'HAP

Le procédé de préparation de l'hydroxyapatite phosphocalcique stœchiométrique Ca₁₀(PO4)₆(OH)₂ consiste en une précipitation lente à haute température, obtenue par double décomposition d'un sel de calcium et d'un sel de phosphore en milieu basique. La réaction est longue, elle se passe à température constante dans un volume réactionnel important et est suivie d'une phase de maturation et d'une phase de lavage à l'eau.

Le précipité ainsi obtenu subit une nouvelle étape de transformation : la séparation solide/solution. Cette dernière étape est réalisée soit par filtration, séchage par étuvage et concassage, soit par atomisation à l'aide d'un lit fluidisé.

Quelle que soit la technique de séparation solide/solution choisie, la poudre va subir deux étapes de transformations spécifiques à l'application de l'invention: l'étape de sélection granulométrique par tamisage à sec pour ne retenir que la tranche granulométrique d'intérêt, inférieure à 25µm ou comprise entre 25 et 45 µm. puis une étape durant laquelle la poudre sera frittée à une température optimale pour assurer une fusion des grains conduisant à des états de surface des poudres bien particuliers, notamment caractérisés par des mesures de surface spécifique tels que définis dans la présente invention, à savoir ≥30 m²/g.

En cours de procédé, des étapes de contrôle de la pureté de la poudre d'HAP sont réalisés par diffraction des rayons X. La largeur à mi-hauteur de certaines raies de diffraction du spectre de diffraction des rayons X de la poudre produite est mesurée, afin de valider le parcours thermique de l'échantillon analysé.

### Paramètres de la synthèse:

- Sels en présence : la réaction chimique est réalisée préférentiellement en mettant en présence une solution de nitrate de calcium tetra hydraté avec une solution de phosphate d'ammonium (spontanément constituée lors du mélange de l'ammoniaque avec l'acide ortho phosphorique). En quantité convenable pour satisfaire à la stœchiométrie de la réaction chimique suivante.

   10 Ca(NO₃)₂, 4H₂O + 6 H₃PO₄ + 20 NH₄OH → Ca₁₀(PO₄)₆(OH)₂ + 20 NH₄NO₃ + 22H₂O
- L'alcalinisation du milieu est obtenue par ajout progressif de solution ammoniacale diluée.
- La température de réaction est maintenue à 70°C +/- 5°C à l'aide d'une double enveloppe dans laquelle circule un fluide caloporteur.
- Le volume réactionnel peut varier de 70 à 150 litres pour le mélange final.
- La durée de la réaction est de 3 à 5h00 en fonction du volume final souhaité.
- La sélection granulométrique est effectuée par tamisage à sec. La poudre retenue est préférentiellement inférieure à 25µm ou comprise entre 25 et 45µm.
- Le frittage est obtenu par une lente montée en température dans un four à moufle. Le rythme de montée en température du four ainsi que la valeur et la durée du plateau seront choisis afin d'obtenir des propriétés particulières de surface spécifique de l'hydroxyapatite, à savoir ≥30 m²/g. De préférence, une montée en température est effectuée pour atteindre une valeur de 600°C (-100°C ; +200°C). La chauffe est arrêtée et une lente descente libre est amorcée jusqu'à un retour à l'ambiante, four fermé.

### Paramètres du contrôle

- La pureté de la poudre d'hydroxyapatite obtenue est contrôlée par diffraction des rayons X, par comparaison aux données publiées dans un référentiel international : le fichier JCPDS (Joint Committee on Powder Diffraction Standards). Un pourcentage de ressemblance du spectre de la poudre obtenue avec les valeurs de diffraction d'une apatite standard répertoriée dans le référentiel JCPDS sous le numéro de fiche 09-0432 est recherché.
- Les impuretés attendues sont le phosphate tricalcique Ca₃(PO₄)₂ et/ ou la chaux CaO. Leur présence est tolérée à hauteur de 5 % environ pour chacune de ces phases.
- La mesure de la largeur à mi-hauteur de la raie majeure en intensité de diffraction de l'hydroxyapatite permet d'avoir des informations sur l'histoire thermique des poudres fabriquées.
- Sur un spectre de diffraction des rayons X, en se plaçant à une valeur d'angle en 2θ d'environ 31.773 degrés +/- 1%, ce qui correspond à la raie de diffraction majeure de l'hydroxyapatite, une mesure de la largeur à mi-hauteur est alors effectuée. La largeur à mi-hauteur à la hauteur totale de la raie à environ 31.773 degrés +/- 1% est alors reportée afin d'obtenir un pourcentage. Plus une hydroxyapatite est traitée thermiquement, plus elle est organisée d'un point de vue cristallographique. Plus les raies de diffraction des rayons X de son spectre sont fines et plus la largeur à mi-hauteur de la raie principale est faible. Cette mesure permet de qualifier l'histoire thermique de la poudre obtenue. Dans le procédé selon l'invention, un rapport de largeur à mi-hauteur sur la hauteur totale égale 0,301%.

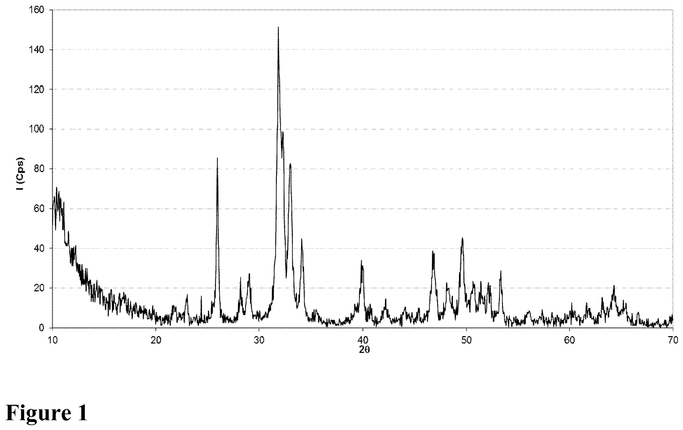

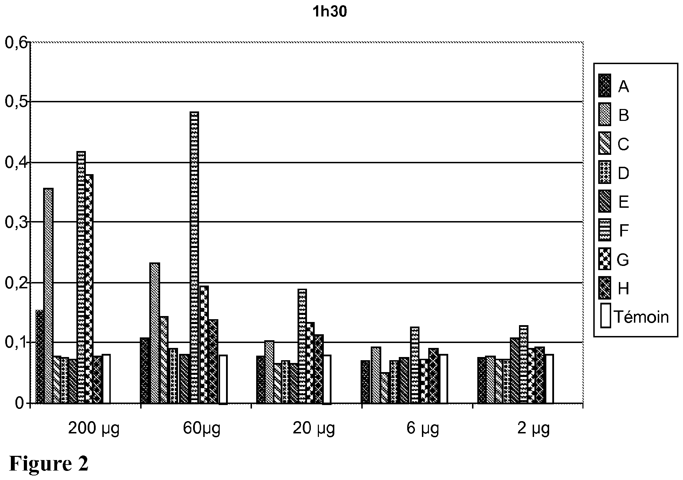

La **figure 1** illustre le spectre de diffraction des rayons X d'une poudre d'hydroxyapatite frittée à 600°C selon l'invention. En abscisses figure 2 téta correspondant à l'angle de diffraction et en ordonnées l'intensité (hauteur) des différents pics.

| | | | | 2 téta | 2 téta | | |
|---|---|---|---|---|---|---|---|
| | 100% | | mi hauteur | borne basse | borne haute | largeur mi hauteur | largeur mi hauteur/hauteur |
| 0 | 31,85 | 142,7 | 71,3 | 31,67 | 32,265 | 0,595 | 0,417% |
| 300 | 31,88 | 134,7 | 73,3 | 31,645 | 32,135 | 0,490 | 0,364% |
| 600 | 31,85 | 152,3 | 76,1 | 31,677 | 32,135 | 0,458 | 0,301% |
| 900 | 31,85 | 270 | 135 | 31,742 | 31,946 | 0,204 | 0,076% |
| 1000 | 31,81 | 332 | 166 | 31,731 | 31,903 | 0,172 | 0,052% |

A l'issue de ce procédé, les caractéristiques suivantes sont observées sur la poudre:
1) les particules ont une granulométrie inférieure à 200µm et la majorité est comprise entre 0 et 25µm ou entre 25 et 45µm ;
2) les particules ont une surface spécifique supérieure à 30 m²/g ;
3) les particules sont sphériques ou de forme irrégulière en fonction des options de fabrication choisies
4) Sur le spectre de diffraction des rayons X dans les conditions du contrôle définies ci-dessus, le rapport de largeur à mi-hauteur sur la hauteur totale est égal à 0.301%.

### Exemple 2 : Procédé de préparation de la composition.

### a) préparation de l'échantillon tumoral

Une fraction d'environ 1cm³ d'une tumeur préalablement prélevée lors d'une biopsie chirurgicale ou d'une biopsie à visée diagnostique à l'aide d'une tréphine, est prélevée de façon stérile puis découpée en petits fragments. Ils sont introduits dans un tube de broyage stérile pré-rempli avec 1g de billes d'alumine de diamètre 1mm. 4 ml d'une solution de Na2CO3 0,03M y sont ajoutés (1V/1V). Le tube est alors placé dans un broyeur pendant 3 minutes. L'opération est répétée jusqu'à obtention d'un broyat liquide. La solution de broyage est transvasée dans un tube de centrifugation à l'aide d'une pipette stérile.

### b) extraction des protéines cytoplasmiques de l'échantillon tumoral

Une biopsie broyée est centrifugée à 6000 tours/minute durant 5 minutes afin d'éliminer les débris membranaires. Le surnageant est conservé, transvasé dans un tube de centrifugation stérile et dilué à 50% (1V/1V) dans une solution sursaturée de sulfate d'ammonium (NH₄)₂SO₄, puis placé à 4°C pendant 1h et enfin centrifugé à 6000 tours/minutes pendant 30 minutes. Le culot remis en suspension dans 1 ml d'une solution de (Na₂HPO₄ ; NaH₂PO₄) à 0,02M à pH 6,8 puis est dilué à 75% (1V/2,3V) dans la même solution de sulfate d'ammonium.

### c) passage des protéines de l'étape b) au travers d'une colonne de chromatographie contenant la poudre d'HAP

La poudre préparée par le procédé de l'exemple 1 est mise dans une colonne de chromatographie dont l'extrémité inférieure est bouchée. Le contenu du tube obtenu à l'issue de l'étape b) est versé dans la colonne de chromatographie et passé à travers la poudre d'hydroxyapatite (phosphate de calcium). L'extrémité supérieure de la colonne est bouchée et la colonne est alors agitée vigoureusement puis mise au repos pendant 3 minutes. Une seringue remplie d'air est adaptée à l'extrémité supérieure de la colonne dont l'extrémité inférieure est débouchée. Une pression lente est exercée sur le piston de la seringue afin de faire passer tout le liquide au travers de la colonne d'HAP ainsi mise en contact avec les protéines de l'échantillon tumoral.

### d) lavage de la poudre issue de l'étape c) ou composition

La colonne est ensuite lavée par une solution de tampon phosphate ou de NaCl à 0,02 M et pH 6.8.

### e) mise en suspension de la composition

Le tampon phosphate est ensuite éliminé par pression à la seringue puis remplacé par la solution d'injection. Après avoir obturé l'extrémité inférieure de la colonne issue de l'étape d), 4 ml d'une solution d'injection constituée de carboxymethyl cellulose à 2% dans une solution de NaCl 0,02M est introduite dans la colonne d'HAP.

La colonne d'HAP chargée des protéines spécifiques de la tumeur (biopsie) est obturée aux deux extrémités puis agitée afin de mettre la poudre d'HAP en suspension dans le liquide d'injection.

Une fois la poudre en suspension, la composition est prête à l'emploi. Il suffira de raccorder des seringues de 1 ml afin de pomper 0,5 ml directement dans la colonne et d'injecter cette dose au patient à traiter.

### Exemple 3 : Kit comprenant de la poudre d'HAP selon l'invention et sa mise en œuvre.

Le kit doit permettre à un praticien en médecine animale ou humaine de purifier les peptides spécifiques d'une tumeur d'un patient et ceci grâce à la mise en contact des poudres d'HAP selon l'invention et de l'échantillon tumoral. Cette poudre d'HAP va fixer ces peptides spécifiques de la tumeur pour être ensuite injectée dans le tissu en sous-cutané ou en intradermique

### Contenu du kit :

| concentration | contenant | Contenu en ml. | composition chimique | désignation |
|---|---|---|---|---|
| 0,03M | 5ml | 4 | Na₂CO₃ ou NaH CO₃ | **I** |
| saturation | 10ml | 8 | (NH₄)₂ SO₄ | **II** |
| 0,02M pH 6,8 | 10ml | 8 | Na₂HPO₄;NaH₂PO₄ | **III** |
| 2% CMC ds NaCl 0,02M | 5ml | 4 | CMC + NaCl | **IV** |

### Mise en œuvre du kit (mode opératoire) :

Les étapes de l'exemple 2 ci-dessus sont mises en œuvre.

Ce kit contient le matériel strictement nécessaire afin d'extraire les protéines cytoplasmiques d'un échantillon tumoral, de purifier les HSPs cellulaires et de les fixer sur les poudres d'HAP et de préparer la suspension à injecter. Il associe de manière originale les solutions salines afin d'extraire les protéines cytoplasmiques et un dispositif fermé permettant de passer de manière stérile et sous pression une solution de protéines à travers une colonne fermée contenant une poudre selon l'invention, biocompatible et injectable, de laver la poudre afin d'éliminer les protéines sans intérêt biologique, de disperser les poudres dans une solution d'injection et de charger des seringues sans ouvrir la colonne. La non-ouverture de la colonne est un avantage considérable car il permet de limiter les risques de contamination.

Le kit contient en outre des seringues de 1ml stériles et des aiguilles stériles. Des étiquettes petit et grand format, des tubes de centrifugation et de prélèvement d'échantillon stériles.

### Exemple 4 : Effet de la poudre selon l'invention sur la stimulation de l'inflamasome.

L'inflammasome est un complexe protéique oligomérique impliqué dans l'immunité innée ou non-spécifique. Il est formé suite à la reconnaissance de divers signaux inflammatoires (LPS, cristaux d'acides uriques, composantes virales et bactériennes diverses) par des protéines de la famille NLPR. L'inflammasome favorise la maturation des cytokines inflammatoires IL-1β et IL-18, en les clivant via l'activation de sa caspase 1. L'inflammasome est responsable de l'activation des processus inflammatoires, et peut induire un phénomène de pyroptose, programme de mort cellulaire différent de l'apoptose. Bien que les cytokines inflammatoires synthétisées au niveau tumoral puissent être impliquées dans le développement de la tumeur, l'activation de l'inflammasome par les adjuvants à distance de la tumeur est indispensable au cross priming des lymphocytes T et au recrutement des APCs.

Plusieurs poudres d'HAP ont été testées sur leur capacité à stimuler l'inflammasome. Les poudres sont utilisées comme deuxième signal (après un ligand (PMA) actif sur des récepteurs TLR) et sont mises au contact de cellules (THP1 d'in vivogen®) qui vont produire de l'ILlb. La mise en contact se fait pendant 1^{1/2}h, 3^{1/2}h, et 7^{1/2}h. Cette IL1b va agir sur une seconde lignée cellulaire qui a un gène de la phosphatase alcaline couplé au facteur de transcription (NF-kB) sur lequel l'IL-lb agit. Après réaction avec son substrat, on dose la phosphatase alcaline en densité optique (DO). L'activation de la phosphatase alcaline est ici un reflet de l'activation du système immunitaire mis en œuvre dans le processus anti-tumoral. Il y a donc une corrélation entre la mesure de l'activité phosphatase alcaline et l'activation de l'inflamasome (NLPR3).

La mesure de la quantité de phosphatase alcaline est faite par spectrophotométrie selon les méthodes classiques.

Les poudres avec les caractéristiques suivantes ont été testées.

| Poudre | Quantité d'HAP (en %) | Forme des particules de la poudre | Granulométrie (en µm) | Température de frittage pour la préparation de la poudre (en °C) | Surface spécifique (en m²/g) |
|---|---|---|---|---|---|
| A | >98 | irrégulière | 0-25 | 900 | 2.9 |
| B | >98 | irrégulière | 0-25 | 500 | 33.53 |
| C | >98 | sphérique | 80-125 | 1180 | 0.6 |
| D | >98 | irrégulière | 80-160 | 1180 | 0.77 |
| E | >98 | sphérique | 22-45 | 1180 | 0.9 |
| F | >98 | sphérique | 0-25 | 600 | 31.11 |
| G | >98 | irrégulière | 0-25 | 600 | 34.69 |
| H | >98 | aciculaire | 0-25 | 0 | 10.11 |

La **figure 2** illustre la mesure de l'activité phosphatase alcaline après 1^{1/2}h de mise en contact de la poudre avec des cellules et ceci en fonction des différentes doses de poudre.

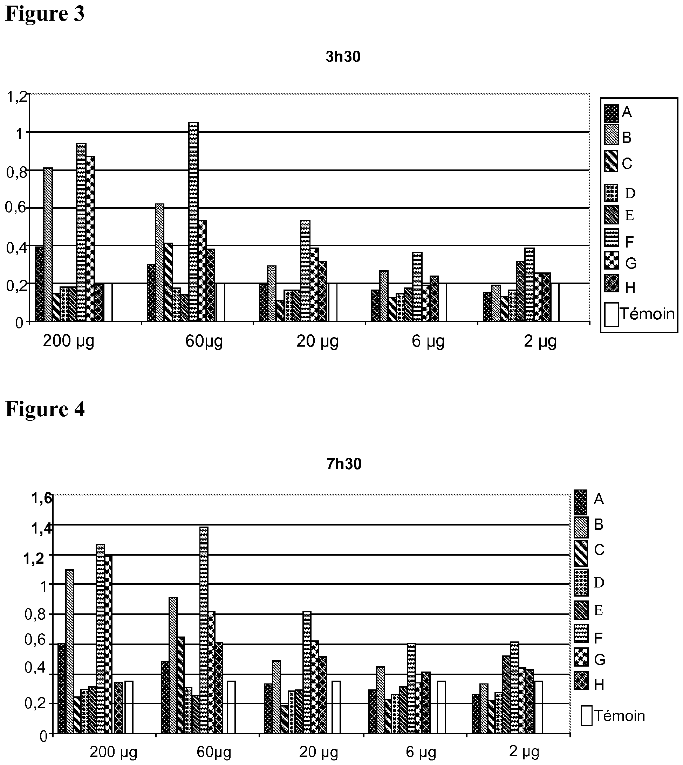

La **figure 3** illustre la mesure de l'activité phosphatase alcaline après 3^{1/2}h de mise en contact de la poudre avec des cellules et ceci en fonction des différentes doses de poudre.

La **figure 4** illustre la mesure de l'activité phosphatase alcaline après 7^{1/2}h de mise en contact de la poudre avec des cellules et ceci en fonction des différentes doses de poudre.

D'après les résultats, il apparaît que les poudres B, F et G permettent une meilleure activation de l'inflammasome, en particulier avec des doses comprises entre 20µl et 200µl. Ces poudres peuvent donc intervenir dans l'activation du système immunitaire cytotoxique via le mécanisme anti-tumoral. Les poudres B, F et G présentent toutes les 3 des caractéristiques communes, à savoir une granulométrie comprise entre 0 et 25µm, une surface spécifique supérieure à 30 m²/g et le frittage est réalisé à une température de 500°C et 600°C.

### Exemple 5 : Effet des protéines désorbées des particules vaccinales préparées selon l'invention et mises au contact sur de cellules RAW 264.7 (ATCC).

Les protéines provenant d'un vaccin préparé comme décrit à partir d'un osteosarcome de chien sont désorbées des particules par une solution de K₂HPO₄. Ces protéines sont marquées par une molécule de biotine à l'aide de sulfo-NHS-biotine. Elles sont ensuite mises en contact avec les cellules RAW en culture après ou pas que les cd91 aient été bloquées ou pas par des anticorps anti-cd91. Les cellules sont ensuite mises en présence d'une streptavidine marquée à la péroxydase et révélée par une diaminobenzidine. Le témoin positif est une gp96 marquée et utilisée de la même manière.

Il apparait que toutes les cellules sont marquées par les protéines purifiées à partir du vaccin ainsi que par les gp96. Dans les deux cas le marquage est inhibé par la mise en contact des cellules au préalable avec un anti cd91. Ceci démontre que les protéines issues du vaccin se fixent sur les cellules présentatrices d'antigènes uniquement via les récepteurs cd91, ce qui correspondrait donc à des protéines de choc thermique (HSPs).

### Exemple 6 : Effet de la composition dans le traitement de l'ostéosarcome chez le chien.

L'ostéosarcome est une tumeur maligne osseuse primaire. Elle se situe de préférence sur les os longs. Les chiens atteints d'ostéosarcome ont un pronostic vital très défavorable. La survie moyenne est d'environ 2 mois. L'amputation et chimiothérapie donnent de meilleurs résultats mais elles ne sont pas toujours possibles.

Sachant que les protéines du choc thermique peuvent s'associer à des peptides synthétisés par les cellules cancéreuses et activer le système immunitaire (via les lymphocytes T et en particulier les CD8), il a été testé, chez des chiens sains et atteints d'ostéosarcome, une méthode de vaccination basée sur l'injection de poudres selon l'invention portant des protéines spécifiques de la tumeur du chien (ce sont principalement les protéines du choc thermique associées à des peptides tumoraux). Il a été mesuré si la vaccination avec ces poudres pouvait avoir un effet clinique sur ces chiens sains et pathologiques.

Selon le procédé décrit dans l'exemple 2, des protéines tumorales provenant de biopsies de chiens atteints d'ostéosarcome ont été adsorbées sur une poudre d'HAP selon l'invention. Huit injections ont été effectuées chez des chiens à différents intervalles de temps. Après cet auto-vaccin, la survie globale et la survie sans progression de l'ostéosarcome ont été mesurées chez chaque chien et comparées aux données de la littérature.

Aucun effet secondaire local ou systémique n'a été mis en évidence chez les chiens après injection. Il est noté que le taux de survie globale chez tous les chiens ayant reçu une injection (sains ou atteints d'un ostéosarcome) est meilleur que la survie globale moyenne des chiens rapportée dans la littérature.

Il est également démontré que la survie sans progression de la maladie est très proche de la survie globale et que la zone d'inflammation de la zone de la tumeur régresse après chaque injection.

Il est observé, aux rayons X, un remodelage de la région de la tumeur après la vaccination.

Ces résultats montrent que les auto-vaccins avec la poudre d'HAP selon l'invention chargée des protéines de choc thermique d'une tumeur du chien (composition selon l'invention) représentent une bonne thérapie adjuvante pour éliminer les cellules disséminées lors de l'évolution d'une tumeur ou cancer et/ou ralentir le développement de métastases.

### Exemple 7 : Effet de la composition versus la chimiothérapie dans le traitement de l'ostéosarcome chez le chien.

22 chiens présentant des ostéosarcomes ont été répartis en 3 groupes différents correspondant à trois traitements différents :
- un premier groupe de chiens (5 chiens) ne reçoit aucun traitement,
- un second groupe de chiens (10 chiens) reçoit le traitement par chimiothérapie seule telle que publiée dans la littérature (Takuo Shida et coll. Low-dose chemotherapy for canine appendicular osteosarcoma, Journal of Japan Veterinary Cancer Society, vol.2, No.1, 1-6 2011)
- un troisième groupe de chiens (7 chiens) est vacciné par la composition selon l'invention telle qu'obtenue à l'issue de l'exemple 2 (poudre d'hydroxyapatite portant des protéines cytoplasmiques de la tumeur du chien à traiter).

La survie globale en jours de l'ensemble des chiens de cette étude a été observée.

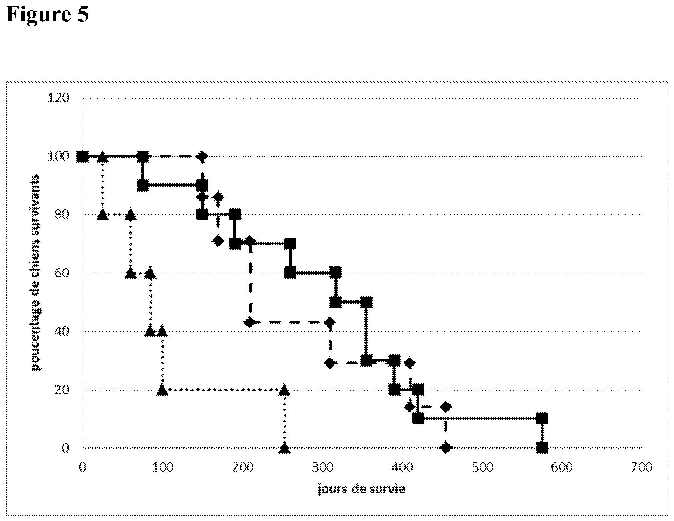

La **figure 5** illustre ces résultats de survie des chiens non traités/ des chiens sous chimiothérapie/ des chiens vaccinés avec la composition selon l'invention.

Les chiens n'ayant reçu aucun traitement ont une survie d'environ 250 jours tandis que les chiens traités par chimiothérapie ont une survie d'environ 580 jours et les chiens traités par immunothérapie ont une survie globale de 450 jours environ. Ceci montre que l'immunothérapie par la composition selon l'invention donne des résultats très satisfaisants et comparables aux résultats obtenus avec une chimiothérapie, tout en ayant moins d'inconvénients que la chimiothérapie qui est très contraignante et a beaucoup d'effets secondaires. Aucun effet secondaire n'a été noté chez les chiens traités par immunothérapie avec la composition selon l'invention.

### Exemple 8 : Effet de la composition selon l'invention dans le traitement de lymphomes B chez le chien.

Le lymphome est un cancer du système lymphatique qui se développe aux dépens des lymphocytes. Il est caractérisé par des proliférations cellulaires malignes dans les organes lymphoïdes secondaires. Parmi les lymphomes malins agressifs, on trouve les lymphomes de phénotype B et de phénotype T, aussi appelés lymphomes B et T.

Une étude de l'effet de la composition selon l'invention sur les lymphomes a été menée sur 50 chiens souffrant de lymphomes B avérés histologiquement.

Les chiens ont été typés suivant les normes OMS :
- Stade I : atteinte d'un ganglion ou d'un organe.
- Stade II : atteinte régionale de plusieurs ganglions avec ou sans les amygdales ;
- Stade III : poly-adénomégalie ;
- Stade IV : atteinte du foie et/ou de la rate en plus des stades I à III ;
- Stade V : atteinte de la moelle osseuse et du sang en plus des stades I à IV.

Les sous- stades (a) correspondant à sans signes cliniques et (b) avec signes cliniques sont également évalués.

Un ganglion est prélevé sur un chien présentant un lymphome B et les doses d'immunothérapie sont préparées suivant le protocole évoqué plus haut avec le procédé de préparation de la composition et le kit de vaccination (cf. exemples 1 à 4).

Une dose est injectée en sous cutané une fois par semaine pendant 4 semaines puis une fois par mois pendant 4 mois. 8 doses sont donc utilisées.

Deux groupes de chiens ont été faits :
a. le premier groupe a reçu ces doses d'immunothérapie associées à un protocole de chimiothérapie incluant asparaginase, vincristine, adriblastine, cyclophosphamide, lomustine et prednisone;
b. le second groupe n'a reçu que les doses d'immunothérapie.

Ces différentes données ont été ensuite comparées aux données de la littérature.

Les chiens (n = 16) à un stade très avancés (Va ou Vb) sont exposés car leur espérance de vie est très courte. Les données de la littérature et les données expérimentales donnent les résultats suivants :

| | Médiane de survie (en jours) | Moyenne de survie (en jours) |
|---|---|---|
| Chiens au stade V (a ou b) non traités | 7 | 43 |
| Chiens au stade V (a ou b) sous chimiothérapie | 36 | 93 |
| Chiens au stade V (a ou b) avec la moelle infiltrée à plus de 30%, non traités | 4 | 4 |
| Chiens au stade V (a ou b) avec moelle infiltrée à 30% sous chimiothérapie | 21 | 30 |
| Chiens au stade V (a ou b) sous chimiothérapie et vaccinés avec le médicament selon l'invention (groupe 1) | >210 | >225 |
| Chiens au stade V (a ou b) vaccinés avec le médicament selon l'invention (groupe 2) | 160 | 208 |

Ainsi, pour le groupe 1 (immunothérapie selon l'invention associée à la chimiothérapie), les données obtenues sont beaucoup plus encourageantes que celles obtenues dans la littérature sans vaccination (médiane à 36 jours et moyenne à 93 jours).

Sur un plan clinique, les chiens du groupe recevant la chimiothérapie et l'immunothérapie sont restés en rémission complète tout le temps de l'expérimentation et ceux recevant l'immunothérapie seule conserve un état stable jusqu'à ce que la maladie reprenne et nécessite leur euthanasie.

Au total, l'immunothérapie associée ou non à la chimiothérapie apporte une espérance de vie significativement plus élevée que lorsqu'elle n'est pas employée. Aucun effet secondaire n'a été constaté.

### Exemple 9 : Effet de l'association médicamenteuse selon l'invention dans le traitement de lymphomes B chez le chien.

Une étude de l'effet de la composition selon l'invention sur les lymphomes a été menée sur 50 chiens souffrant de lymphomes B avérés histologiquement.

Les chiens ont été typés suivant les normes OMS :
- Stade I : atteinte d'un ganglion ou d'un organe.
- Stade II : atteinte régionale de plusieurs ganglions avec ou sans les amygdales ;
- Stade III : poly-adénomégalie ;
- Stade IV : atteinte du foie et/ou de la rate en plus des stades I à III ;
- Stade V : atteinte de la moelle osseuse et du sang en plus des stades I à IV.

Les sous- stades (a) équivalent à sans signes cliniques et (b) avec signes cliniques sont également évalués.

Un ganglion est prélevé sur un chien présentant un lymphome B et les doses d'immunothérapie sont préparées suivant le protocole évoqué plus haut avec le procédé de préparation de la composition et le kit de vaccination (cf. exemples 1 à 4).

Une dose préparée selon l'exemple 2 est injectée en sous cutané une fois par semaine pendant 4 semaines puis une fois par mois pendant 4 mois. 8 doses sont donc utilisées. Le vaccin est préparé à partir d'une biopsie chirurgicale ganglionnaire.

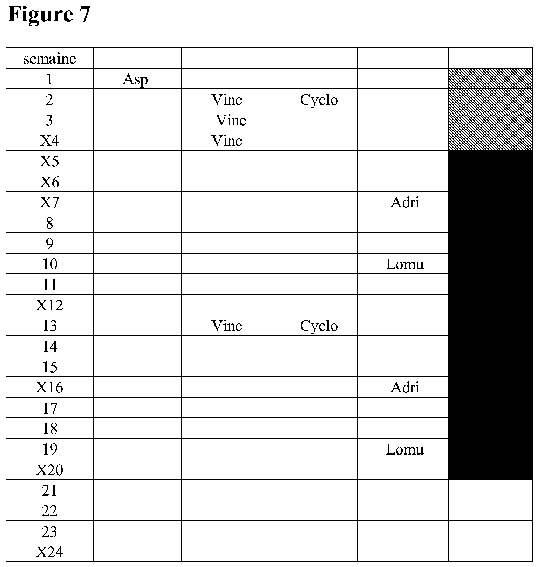

Trois groupes de chiens ont été faits :
- un premier groupe pour lequel ces doses ont été associées à un protocole de chimiothérapie incluant de l'asparaginase, de la vincristine, adriblastine, cyclophosphamide, lomustine et prednisone administré suivant le protocole illustré en **figure 7**,
- un second groupe a reçu la chimiothérapie et seulement des doses de placebo ne contenant pas de protéines autologues (composition selon l'invention),

A T0 et à la fin du traitement une injection intradermique (0.1ml) d'extrait tumoral autologue a été effectué afin de vérifier si une réaction d'hypersensibilité retardée (48-72h) survenait.
- un troisième groupe a reçu les doses de vaccin seules.

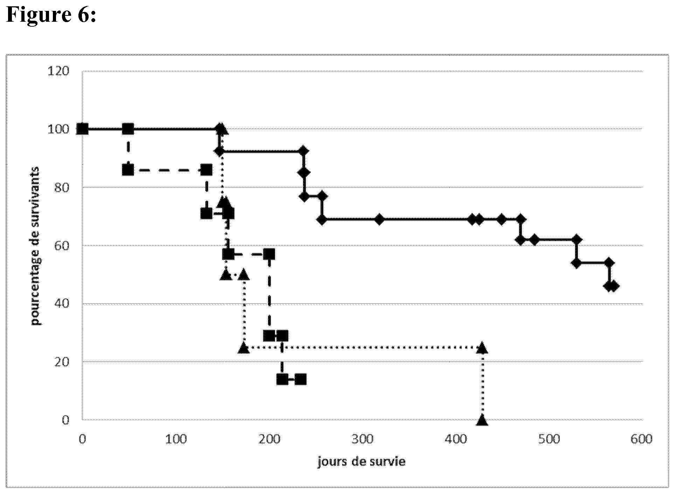

La **figure 6** illustre les résultats de l'étude. Il s'agit de trois courbes montrant la survie en jours et le pourcentage de survie des chiens atteints de lymphome B traités par l'auto-vaccin selon présente invention seul (-▲-), des chiens traités par l'auto-vaccin selon présente invention en association avec une chimiothérapie (-◆-) et des chiens traités par un placebo en association avec une chimiothérapie (-■-).

Les courbes de survie montrent une survie très significativement augmentée lorsque les chiens reçoivent un traitement associant chimiothérapie et immunothérapie (la composition selon l'invention) par rapport à ceux ne recevant que les doses de chimiothérapie. Le groupe recevant uniquement l'immunothérapie a une survie comparable à celui recevant la chimiothérapie seule

Au total, l'immunothérapie associée ou non à la chimiothérapie apporte une espérance de vie significativement plus élevée que lorsqu'elle n'est pas employée. Aucun effet secondaire n'a été constaté.

Ces résultats suggèrent que cette technique de stimulation du système immunitaire est efficace et pourrait présenter un intérêt fort chez l'homme en association avec un traitement chimio/radio-thérapeutique.

Lors de rechute, les chiens ont reçu une chimiothérapie dite de secours identique. Trois d'entre eux ont en plus bénéficié d'une revaccination par des vaccins faits à partir de la biopsie initiale alors que 4 autres chiens ont bénéficié d'une revaccination par un vaccin fait à partir d'une nouvelle biopsie faite à la rechute. Les deux groupes ont eu une survie plus importante que les chiens ayant reçu la chimiothérapie de secours seule. Le groupe des chiens vaccinés à partir de la nouvelle biopsie a une survie plus longue que ceux des chiens vaccinés à partir de la biopsie initiale. Il existe donc un intérêt lors de rechute à faire un nouveau vaccin à partir d'une nouvelle biopsie.

### Exemple 10 : Effet de l'utilisation de la composition dans le traitement du mélanome chez le cheval.

Les mélanomes équins sont beaucoup plus fréquents chez les chevaux gris et blancs que chez les chevaux ayant une robe sombre. Certaines races sont particulièrement exposées comme les camarguais ayant une prévalence de près de 70% après 15 ans.

Les localisations les plus communes et les plus typiques de ces tumeurs sont la face ventrale de la queue (94% des cas chez le cheval camarguais), le périnée (43%) et les organes génitaux externes.

Une étude de Seltenhammer et al très intéressante sur 296 chevaux gris (Lipizzan) a montré que 148 d'entre eux manifestaient des mélanomes dermiques (50%). Des 68 ayant plus de 15 ans, 51 avaient un mélanome. Dans 75% des cas les mélanomes ont été détectés sous la queue.

On peut distinguer plusieurs types d'évolution de ces tumeurs:
- une croissance lente sur de nombreuses années (jusqu'à 20 ans) sans métastases;
- une croissance lente sur plusieurs années suivie par une croissance soudainement rapide et une transformation maligne ;
- une croissance rapide et maligne d'emblée.

Il n'existe pas de véritable traitement de ces tumeurs autres que la résection chirurgicale qui est souvent délicate en raison de leur localisation, de leur multiplicité et de leur réapparition quasi systématique.

Il n'est pas envisageable une quelconque chimiothérapie chez le cheval en raison de la faiblesse du rapport bénéfice/risque et des contraintes techniques que cela engendrerait. La composition selon l'invention telle que décrite La stimulation du système immunitaire contre les protéines anormales synthétisées par les cellules tumorales via la composition selon l'invention et telle que décrite dans l'exemple 2 est une alternative intéressante, et d'autant plus qu'elle n'entraîne pas ou peu d'effets secondaires. De plus, la mise en œuvre est compatible avec le maintien de l'animal dans son environnement habituel.

Les protéines anormales des cellules cancéreuses du mélanome du cheval ont été adsorbées sur une poudre d'hydroxyapatite selon la technique décrite dans l'exemple 2 (adjuvant minéral de vaccination) avec le kit décrit et ceci afin de stimuler le système immunitaire de l'animal à partir duquel les cellules cancéreuses ont été isolées.

Une tumeur de forme ovoïde du cheval a été choisie comme témoin car facilement mesurable.

Le vaccin consiste en une injection de 0,5ml de la composition (poudre d'hydroxyapatite chargée des protéines spécifiques de la tumeur du cheval) dans le tissu sous cutané toutes les semaines pendant 1 mois et tous les mois pendant 4 mois, ce qui fait un total de 8 injections.

Il n'a été noté aucun effet secondaire, ni local, ni systémique, le cheval étant resté au champ pendant toute la durée du traitement et ayant eu une activité normale.

Le volume de la tumeur calculé à partir des mesures effectuées a réduit de 70% en un peu moins de 3 mois.

Cette étude montre que cette méthode de traitement décrit ici est efficace sur le mélanome du cheval et permet une régression de la taille de la tumeur. Il peut être envisagé une rémission totale.

### Exemple 11 : Effet de la réutilisation de la composition lors d'une récurrence de lymphome B après rémission complète

Sur une série de 5 chiens qui ont été victimes à des temps variable d'une récurrence de leur lymphome B après une rémission clinique et biologique complète, se manifestant par une réapparition de ganglions palpables et un grossissement des tumeurs de divers organes identifiées dans le bilan d'extension initial ou la réapparition d'un envahissement médullaire, un traitement suivant l'invention a été refait à partir d'une biopsie ganglionnaire initiale ou prélevée après la récurrence. Deux groupes ont été différenciés : un groupe ayant reçu la chimiothérapie plus un traitement selon l'invention et traité à nouveau selon l'invention après récurrence avec la biopsie initiale (I), un second groupe ayant reçu la chimiothérapie plus un traitement selon l'invention et traité à nouveau selon l'invention après récurrence avec la deuxième biopsie (post récurrence) (II). La survie après récurrence de ces deux groupes a été comparée à celle des chiens placebo traités avec un nouveau cycle de chimiothérapie après récurrence (T). Il apparait que la survie des deux groupes testés (I et II) est plus importante que celle du groupe T et que la survie du groupe II est plus importante que celle du groupe I. Il existe donc un intérêt à traiter à nouveau selon l'invention après récurrence de la maladie et si possible à partir d'une nouvelle biopsie.

### Exemple 12 : Synthèse d'interféron gamma par des lymphocytes T stimulés par des cellules dendritiques activées in vitro par la poudre utilisée suivant l'invention.

Le kit suivant l'invention a été utilisé pour purifier des protéines d'une tumeur obtenue par greffe de cellules RT-4T1 sur des souris balb/c. Des cellules dendritiques de ces souris sont obtenues à partir de macrophages cultivés en présence d'Il-4 et de GM-CSF. A cinq jours de cultures, ces cellules sont mises en présence de quantités variables de solution pour 106 cellules pendant 48 heures. Ces cellules sont mises ensuite au contact de lymphocytes T de la même lignée de souris, l'interféron gamma synthétisé par ces cellules est dosé par test Elisa. A partir de 20 µl de solution de 106 cellules, on a une synthèse significative d'interféron gamma par rapport au témoin négatif.

## Revendications

1. Composition pour son utilisation comme auto-vaccin anti-tumoral pour le traitement d'un lymphome B ou T chez un sujet, comprenant une poudre d'hydroxyapatite et/ou de phosphate tricalcique sur laquelle sont adsorbées des protéines cytoplasmiques obtenues à partir d'un échantillon tumoral du sujet, ladite poudre présentant une surface spécifique SS telle que SS ≥30 m²/g et une granulométrie G telle que G ≤ 200µm.

2. Composition pour son utilisation selon la revendication 1, dans laquelle la poudre a subi une étape de frittage comprise entre 400 et 600°C.

3. Composition pour son utilisation selon la revendication 1 ou 2, dans laquelle la poudre présente une granulométrie G telle que G ≤ 25µm.

4. Composition pour son utilisation selon la revendication 1 ou 2, dans laquelle la poudre présente une granulométrie G comprise entre 25µm et 45µm.

5. Composition pour son utilisation selon l'une quelconque des revendications 1 à 4, dans laquelle le spectre de diffraction des rayons X de la poudre présente un rapport de la largeur à mi-hauteur sur la hauteur totale de la raie sur un spectre de diffraction des rayons X, en se plaçant à une valeur d'angle en 2θ égale à 31.773 degrés +/- 1%, reporté en pourcentage, compris entre 0,2% et 0,35%.

6. Composition pour son utilisation selon l'une quelconque des revendications 1 à 5, dans laquelle la poudre est en suspension dans un liquide d'injection.

7. Composition pour son utilisation selon l'une quelconque des revendications 1 à 6, dans laquelle le sujet est un homme.

8. Composition pour son utilisation selon l'une quelconque des revendications 1 à 6, dans laquelle le sujet est un chien.

9. Composition pour son utilisation selon l'une quelconque des revendications 1 à 8, pour son utilisation thérapeutique en association avec un agent anti-tumoral et/ou un agent radio-thérapeutique.

10. Composition pour son utilisation selon l'une quelconque des revendications 1 à 9, **caractérisée en ce qu'**une dose administrée par injection chez le sujet, comprend entre 30 et 50 mg d'hydroxyapatite et entre 1000 et 2000 µg de protéines.

## Patentansprüche

1. Zusammensetzung zur Verwendung als Anti-Tumor-Autovakzine für die Behandlung eines B- oder T-Lymphoms eines Subjekts, umfassend ein Hydroxylapatit- und/oder Trikalziumphosphat-Pulver mit darauf adsorbierten zytoplasmatischen Proteinen, die aus einer Tumorprobe des Subjekts erhalten wurden, wobei das Pulver eine spezifische Oberfläche SS ≥ 30 m²/g und eine Granulometriegröße G ≤ 200 µm aufweist.

2. Zusammensetzung zur Verwendung nach Anspruch 1, wobei das Pulver einen Sinterschritt bei 400 bis 600°C durchlaufen hat.

3. Zusammensetzung zur Verwendung nach Anspruch 1 oder 2, wobei das Pulver eine Granulometrie G ≤ 25 µm aufweist.

4. Zusammensetzung zur Verwendung nach Anspruch 1 oder 2, wobei das Pulver eine Granulometrie G von 25µm bis 45µm aufweist.

5. Zusammensetzung zur Verwendung nach einem der Ansprüche 1 bis 4, wobei das Röntgendiffraktionsspektrum des Pulvers ein Verhältnis der halben Breite zur vollen Höhe des Scheitels in einem Röntgendiffraktionsspektrum bei einem Winkelwert bei 2θ gleich 31.773 Grad +/- 1%, angegeben als Prozentsatz, zwischen 0,2% und 0,35% aufweist.

6. Zusammensetzung zur Verwendung nach einem der Ansprüche 1 bis 5, wobei das Pulver in einer Injektionsflüssigkeit suspendiert ist.

7. Zusammensetzung zur Verwendung nach einem der Ansprüche 1 bis 6, wobei das Subjekt ein Mensch ist.

8. Zusammensetzung zur Verwendung nach einem der Ansprüche 1 bis 6, wobei das Subjekt ein Hund ist.

9. Zusammensetzung zur Verwendung nach einem der Ansprüche 1 bis 8, zur therapeutischen Verwendung in Kombination mit einem Antitumormittel und/oder einem strahlentherapeutischen Mittel.

10. Zusammensetzung zur Verwendung nach einem der Ansprüche 1 bis 9, **dadurch gekennzeichnet, dass** eine dem Subjekt durch Injektion verabreichte Dosis zwischen 30 und 50 mg Hydroxylapatit und zwischen 1000 und 2000 µg Proteine enthält.

## Claims

1. Composition for use as antitumor auto-vaccine for the treatment of B or T lymphomas in a subject, comprising a hydroxyapatite and/or tricalcium phosphate powder on which cytoplasmic proteins from a tumor sample of the subject are absorbed, said powder having a specific surface area SS ≥ 30 m²/g and a particle size G ≤ 200 µm.

2. Composition for use according to claim 1, wherein the powder has undergone a sintering step between 400°C and 600°C.

3. Composition for use according to claim 1 or 2, wherein the powder has a particle size G ≤ 25 µm.

4. Composition for use according to claim 1 or 2, wherein the powder has a particle size G between 25 µm and 45 µm.

5. Composition for use according to any one of claims 1 to 4, wherein the X-ray diffraction spectrum of the powder has a ratio of the width at half maximum to total height of the line on an X-ray diffraction spectrum, with an angle 2θ value equal to 31.773 degrees +/- 1%, reported as percentage, of between 0.2% and 0.35%.

6. Composition for use according to any one of claims 1 to 5, wherein the powder is suspended in an injection liquid.

7. Composition for use according to any one of claims 1 to 6, wherein the subject is a human.

8. Composition for use according to any one of claims 1 to 6, wherein the subject is a dog.

9. Composition for use according to any one of claims 1 to 8, for therapeutic use in combination with an antitumor agent and/or a radiotherapeutic agent.

10. Composition for use according to any one of claims 1 to 9, wherein one dose administered by injection to the subject comprises between 30 mg and 50 mg of hydroxyapatite and between 1000 µg and 2000 µg of proteins.
